# EUROPEAN PATENT SPECIFICATION

(11) **EP 1 411 880 B1**
(45) Date of publication and mention of the grant of the patent: **25.04.2018**
(21) Application number: 02749985.4
(22) Date of filing: 11.07.2002
(51) Int. Cl.: A61K 38/45, C12N 15/86, A61P 35/00

(54) **RECOMBINANT VSV FOR THE TREATMENT OF TUMOR CELLS**
REKOMBINANTE VSV ZUR BEHANDLUNG VON TUMORZELLEN
VSV RECOMBINANT POUR LE TRAITEMENT DE CELLULES TUMORALES

(30) Priority: 11.07.2001 US 304125 P
(43) Date of publication of application: 28.04.2004
(73) Proprietor: University of Miami, Miami, FL 33136 (US)
(72) Inventor: BARBER, Glen, Miami, FL 33176 (US)
(74) Representative: J A Kemp
(86) International application number: PCT/US2002/022146
(87) International publication number: WO 2003/005964

(56) References cited:
- WO-A2-01/19380
- US-B1- 6 168 943
- US-B1- 6 217 860
- Bradley D Howard ET AL: "Transduction of human pancreatic tumor cells with vesicular stomatitis virus G-pseudotyped retroviral vectors containing a herpes simplex virus thymidine kinase mutant gene enhances bystander effects and sensitivity to ganciclovir", Cancer Gene Therapy, vol. 7, no. 6, 1 January 2000 (2000-01-01) , pages 927-938, XP055259784, DOI: 10.1038/sj.cgt.7700180

## Description

### FIELD OF THE INVENTION

The present invention generally relates to vesicular stomatitis virus (VSV), methods of producing heterologous proteins in recombinant VSV and the use of recombinant VSV comprising cytokines or suicide genes for the treatment of malignant cells.

### BACKGROUND OF THE INVENTION

Vesicular stomatitis virus (VSV), of the genus, *Vesiculovirus,* is the prototypic member of the family *Rhabdoviridae,* and is an enveloped virus with a negative stranded RNA genome that causes a self-limiting disease in live-stock and is essentially non-pathogenic in humans. Balachandran and Barber (2000, IUBMB Life 50: 135-8). Rhabdoviruses have single, negative-strand RNA genomes of 11,000 to 12,000 nucleotides (Rose and Schubert, 1987, Rhabdovirus genomes and their products, in The Viruses: The Rhabdoviruses, Plenum Publishing Corp., NY, pp. 129-166). The virus particles contain a helical, nucleocapsid core composed of the genomic RNA and protein. Generally, three proteins, termed N (nucleocapsid, which encases the genome tightly), P (formerly termed NS, originally indicating nonstructural), and L (large) are found to be associated with the nucleocapsid. An additional matrix (M) protein lies within the membrane envelope, perhaps interacting both with the membrane and the nucleocapsid core. A single glycoprotein (G) species spans the membrane and forms the spikes on the surface of the virus particle. Glycoprotein G is responsible for binding to cells and membrane fusion. The VSV genome is the negative sense (i.e., complementary to the RNA sequence (positive sense) that functions as mRNA to directly produce encoded protein), and rhabdoviruses must encode and package an RNA-dependent RNA polymerase in the virion (Baltimore et al., 1970, Proc. Natl. Acad. Sci. USA 66: 572-576), composed of the P and L proteins. This enzyme transcribes genomic RNA to make subgenomic mRNAs encoding the 5-6 viral proteins and also replicates full-length positive and negative sense RNAs. The genes are transcribed sequentially, starting at the 3' end of the genomes.

The sequences of the VSV mRNAs and genome is described in Gallione et al. 1981, J. Virol. 39:529-535; Rose and Gallione, 1981, J. Virol. 39:519-528; Rose and Schubert, 1987, Rhabdovirus genomes and their products, p. 129-166, in R. R. Wagner (ed.), The Rhabdoviruses. Plenum Publishing Corp., NY; Schubert et al., 1985, Proc. Natl. Acad. Sci. USA 82:7984-7988. WO 96/34625 published November 7, 1996, disclose methods for the production and recovery of replicable vesiculovirus. United States Patent No. 6,168,943, issued January 2, 2001, describes methods for making recombinant vesiculoviruses.

Although most immortilized tissue culture cell lines are permissive to VSV, the virus is sensitive to the antiviral actions of the interferons (IFN). Balachandran and Barber, *supra.* Primary cells containing PKR and a functional INF system are not strongly permissive to VSV replication. Balachandran et al. (2000, Immunity 13, 129-141) disclose that mice lacking the IFN-inducible double stranded RNA-dependent protein kinase (PKR), are susceptible to VSV infection. That VSV is capable of replicating in a majority of mammalian cell lines, but not well in primary cells unless PKR function or INF signaling is defective, implies that host defense mechanisms required to prevent VSV replication are impaired in cells permissive to the virus, including immortilized and malignant cells. Balachandran and Barber, *supra.*

VSV oncolytic activity is disclosed in WO 00/62735, published October 26, 2000; Balachandran and Barber, *supra;* Stojdl et al. (2000, Nature Medicine, vol 6, pages 821-825); Balachandran et al. (2001, J. of Virol. p.3473-3479); WO 01/19380, published March 22, 2001; WO 99/18799 published April 22, 1999; and WO 00/62735 published October 26, 2000.

There remains a need for the development of compositions and methods for the treatment of tumor cells.

### BRIEF SUMMARY OF THE INVENTION

The invention is defined by the claims.

The present invention relates to recombinant vesicular stomatitis virus (VSV) expression constructs (vectors) based on VSV which confer infectivity, replication, transcription, or any combination thereof when the construct is introduced into cells either *in vitro* or *in vivo,* with or without a viral particle. The VSV construct is engineered to express one or more heterologous nucleotide sequence(s), especially genes encoding a cytokine, such as for example, interferon or interleukin, or other biologically active molecules, such as for example, heat shock protein gp96, or suicide cassette such as thymidine kinase (TK) or cytosine deaminase. The VSV vector may comprise nucleic acid encoding two or more biologically active proteins, for example, two cytokines, such as an interferon and an interleukin, or two interferons or two interleukins. The two or more cytokines maybe identical or different. The recombinant VSV can be replication-competent or replication-defective. The present invention also relates to methods for producing oncolytic activity in tumor and/or malignant cells comprising administering recombinant VSV vectors comprising nucleic acid encoding a cytokine, including for example, interferon, such as, interferon-beta or interferon-gamma, and interleukin, such as, interleukin 4 or interleukin 12 to the tumor and/or malignant cells.

The present invention provides recombinant vesicular stomatitis virus (VSV) vectors comprising nucleic acid encoding a cytokine(s), wherein said recombinant VSV vector exhibits greater oncolytic activity against a tumor cell than a wild-type VSV vector when contacted with the tumor cell. In some genes encoding a cytokine, such as for example, interferon or interleukin, or other biologically active molecules, such as for example, heat shock protein gp96, or suicide cassette such as thymidine kinase (TK) or cytosine deaminase. The VSV vector may comprise nucleic acid encoding two or more biologically active proteins, for example, two cytokines, such as an interferon and an interleukin, or two interferons or two interleukins. The two or more cytokines maybe identical or different. The recombinant VSV can be replication-defective. The present invention also relates to methods for producing oncolytic activity in tumor and/or malignant cells comprising administering recombinant VSV vectors comprising nucleic acid encoding a cytokine, including for example, interferon, such as, interferon-beta or interferon-gamma, and interleukin, such as, interleukin 4 or interleukin 12 to the tumor and/or malignant cells.

The present invention provides replication-defective recombinant vesicular stomatitis virus (VSV) vectors comprising nucleic acid encoding a cytokine(s), wherein said recombinant VSV vector exhibits greater oncolytic activity against a tumor cell than a wild-type VSV vector when contacted with the tumor cell. In some examples, the cytokine is an interferon, such as interferon-beta or interferon-gamma. In other examples, the cytokine is an interleukin such as for example, IL-4 or IL-12. In some examples, the VSV vector comprises nucleic acid encoding two or more cytokines, such as two interferons or two interleukins or an interferon, such as interferon-beta and an interleukin, such as interleukin-12. The two or more cytokines may be identical or different. In additional examples, the VSV vector lacks G-protein function and may also lack M and/or N protein function(s). In other examples, the tumor cell is a melanoma tumor cell, mammary tumor cell, prostate tumor cell, cervical tumor cell, hematological-associated tumor cell or a cell harboring a defect in a tumor suppressor pathway. In further examples, the tumor cell is a mammalian, such as a human, tumor cell. The present invention also provides viral particles comprising a VSV vector of the present invention.

The present invention also comprises isolated nucleic acid encoding a recombinant VSV vector of the present invention as well as host cells comprising a recombinant VSV vector of the present invention. The present invention also provides methods for making a recombinant VSV vector of the present invention comprising growing a cell comprising said VSV vector under conditions whereby VSV is produced; and optionally isolating said VSV. In some examples, the VSV vector is replication defective and the host cells comprise the VSV protein function essential for VSV replication such that said VSV vector is capable of replication in said host cell. In some examples, the VSV vector comprises nucleic acid encoding in addition to a cytokine, such as an interferon or interleukin; a suicide gene, such as thymidine kinase or cytosine deaminase or other biological protein, such as a heat shock protein, such as for example, gp96.

The present invention also provides compositions comprising a VSV vector or viral particle of the present invention and a pharmaceutically acceptable excipient.

In some examples, the VSV vector is present in the composition in an amount effective to produce oncolytic activity in a tumor cell when said composition is contacted with the tumor cell.

The present invention also provides the use of a VSV vector of the invention in the preparation of a medicament for treating a tumor. In some examples, the VSV vector lacks G-protein function. In yet further examples, the cytokine is an interferon, such as for example, interferon-beta or interferon-gamma; or a cytokine, such as for example, an interleukin, such as interleukin-4 or interleukin-12. In additional examples, the tumor includes a melanoma tumor, mammary tumor, prostate tumor, cervical tumor, hematological-associated tumor or tumor comprising cells harboring defects in a tumor suppressor pathway. In yet further examples, said medicament is for administration by intravenous injection or intratumoral injection.

The present invention also provides the use of a replication-incompetent recombinant VSV vector comprising nucleic acid encoding a suicide gene wherein said VSV vector exhibits greater tumor suppression or oncolytic activity when administered along with a prodrug than a wild-type VSV vector in the preparation of a medicament for treating a tumor. In some examples, the suicide gene encodes thymidine kinase (TK) and the prodrug is ganclyclovir or acyclovir. In other examples, the suicide gene encodes a cytosine deaminase and the prodrug is 5-fluorocytosine. In some examples, the VSV vector lacks G-protein function. In other examples, the tumor is a melanoma tumor, mammary tumor, prostate tumor, cervical tumor, hematological-associated tumor or tumor comprising cells harboring a defect in a tumor suppressor pathway. In other examples, the medicament is for administration by intravenous injection or intratumoral injection.

The present invention also provides the use of tumor cells infected with or lysed by a VSV vector comprising nucleic acid encoding a cytokine, chemokine or heat shock protein in the manufacture of a medicament for treating a tumor. In some examples, the cytokine is an interferon, such as interferon-beta or interferon-gamma, or interleukin, such as interleukin-4 or interleukin-12. The present invention also provides a composition for inducing an immune response in an individual comprising tumor cells infected with or lysed by a VSV vector comprising nucleic acid encoding a cytokine, chemokine or heat shock protein and a pharmaceutically acceptable excipient. The present invention also provides a method for preparing such a composition comprising contacting a tumor cell obtained from an individual with a VSV vector comprising nucleic acid encoding a cytokine, chemokine or heat shock protein under conditions suitable for lysing said tumor cells.

The present invention also provides kits comprising a VSV vector comprising nucleic acid encoding a cytokine or a thymidine kinase and instructions for use of the VSV vector.

Also disclosed are methods of making and using VSV constructs to express a cytokine or other biologically active molecule, such as thymidine kinase or cytosine deaminase, as well as viruses or mammalian cells comprising the VSV expression construct. The present invention also discloses methods of producing difficult to make, toxic or rare proteins. The present invention also discloses methods for producing eukaryotic proteins using VSV expression systems which provide authentic (i.e. eukaryotic) processing.

### BRIEF DESCRIPTION OF THE DRAWING(S)

Figures 1A-1B illustrate the generation of recombinant VSV expressing TK, IL-4, IFN or green fluorescent protein (GFP).
Figure 1A. cDNA representing the VSV genome (pVSV-XN2), flanked by the T7 RNA polymerase leader and T7 terminator as well as hepatitis virus delta ribozyme (RBZ) was used to create recombinant viruses. IL-4, TK or GFP were inserted between the G and L genes of VSV.
Figure 1B. Growth curves of recombinant viruses. BHK cells were infected with wild-type (WT) VSV, VSV-IL-4 and VSV-TK at an m.o.i. of 10. Supernatants from infected cells were harvested at the indicated times post-infection and viral titers determined by plaque assay.
Figures 2A-2D illustrate expression of IL-4 or TK from rVSV.

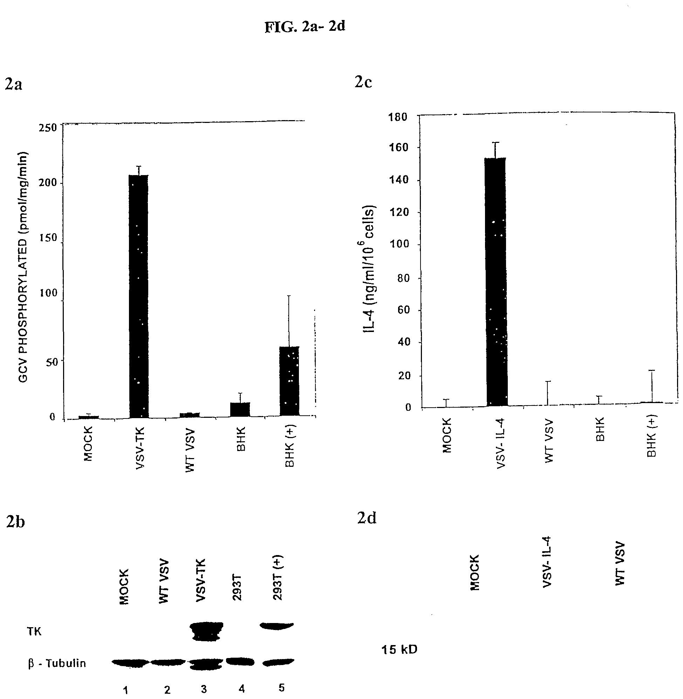

Figure 2A. GCV is phosphorylated in cells infected with VSV-TK. BHK cells were mock infected or infected with VSV-TK or WT VSV (m.o.i.=1) for 8 h and cell lysates were assayed for GCV phosphorylation, *in vitro.* BHK cells transiently transfected with CMV promoter driven HSV-TK [BHK(+)] or empty vector (BHK) were used as controls
Figure 2B. Expression of HSV-TK in VSV-TK infected cells. BHK cells were mock infected (lane 1) or infected with WT VSV (lane 2) or VSV-TK (lane 3), at an m.o.i. of 1, for 24 h and cell extracts analyzed for TK expression using an anti-TK monoclonal antibody. 293T cells transiently transfected with an empty vector (lane 4) or CMV promoter driven HSV-TK (lane 5) were used as a positive control.
Figure 2C. High level expression of IL-4 in cells infected with VSV-IL-4. Culture medium from BHK cells infected with WT VSV or VSV-IL-4 was measured for functional IL-4 using capture ELISA. As further controls, IL-4 was measured in culture medium from BHK cells transiently transfected with an empty vector or CMV-promoter driven IL-4 cDNA.
Figure 2D. Immunoprecipitation of IL-4 from supernatants of VSV-IL-4 infected cells. Extracts from [³⁵S]methionine-labeled cells mock infected (lane 1) or infected with VSV-IL-4 (lane 2) or WT VSV (lane 3) were immunoprecipitated with an IL-4 antibody.

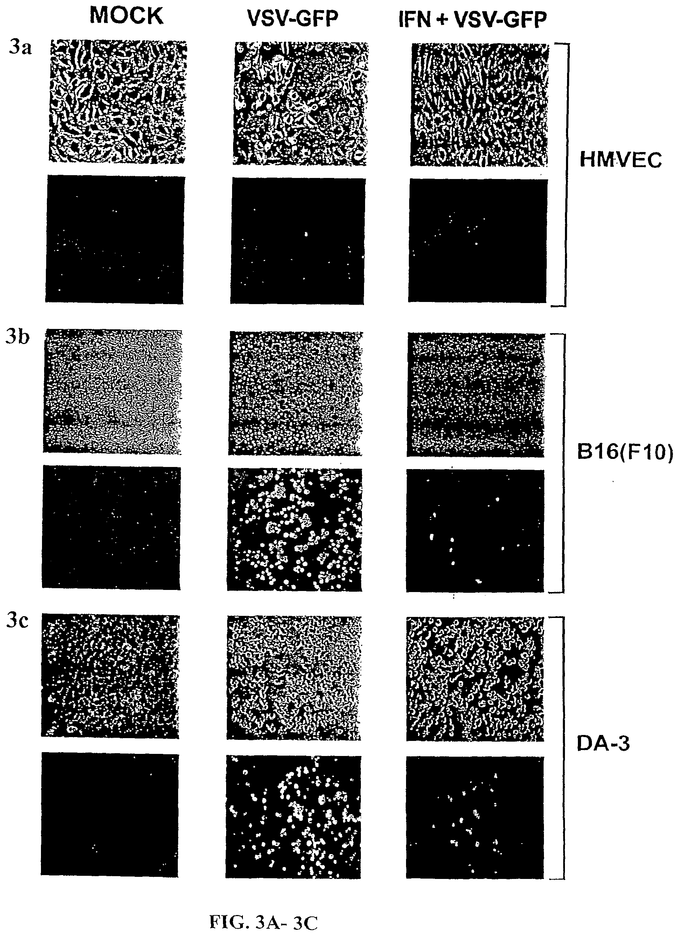

Figures 3A-3F illustrate the *in vitro* effects of wild type and recombinant VSVs on primary or transformed cells.
Figures 3A-3C illustrate efficient replication of VSV-GFP in transformed cells. HMVEC, B16(F10) or DA-3 cells were infected with VSV-GFP with or without prior treatment of IFNα (500 u/ml). Top panels show cells under brightfield microscopy (magnification, 20x) and lower panels shows the same field by immunofluorescence.

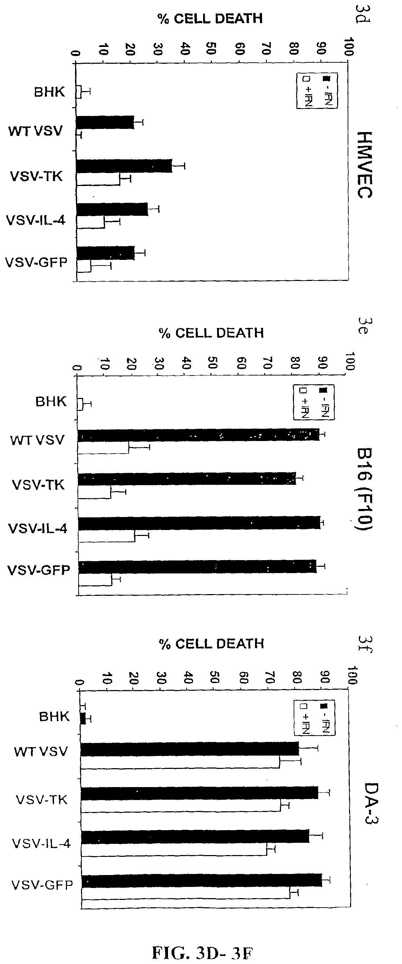

Figures 3D-3F illustrate that rVSVs efficiently kill transformed cells. HMVEC, B16(F10) or DA-3 cells were infected with WT VSV, VSV-TK or VSV-IL-4 with (solid columns) or without (clear columns) prior treatment with IFNα. Cell viability was assayed by Trypan Blue exclusion 18 h after infection.

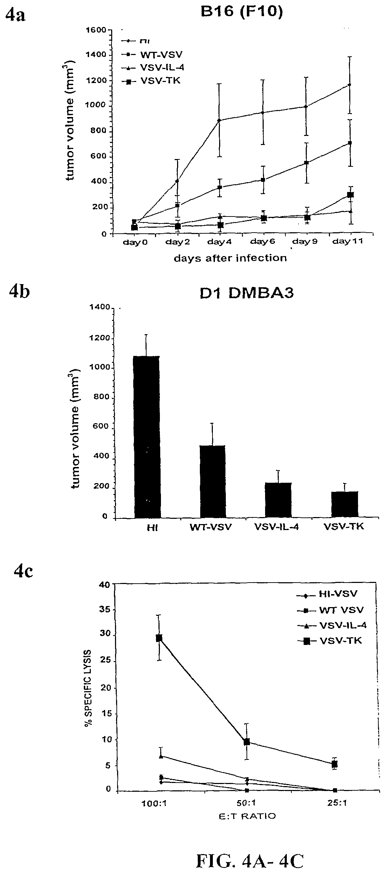

Figure 4A-4C illustrate that rVSV expressing TK and IL-4 inhibit the growth of syngeneic breast and melanoma tumors in immunocompetent mice.
Figure 4A. C57B1/6 mice were implanted subcutaneously with 5x10⁵ B16(F10) melanoma cells. After palpable tumors had formed, animals were treated intratumorally with 2x10⁷ p.f.u. WT VSV, VSV-IL-4 or VSV-TK. Injections of virus were repeated after 3 days. Tumor volumes were calculated and are shown as a mean + S.E.M. (n=5). Two mice that received heat inactivated virus were sacrificed at day 4 due to the large size of tumors.
Figure 4B. BALB/c mice were implanted subcutaneously with 1.5x10⁶ D1 DMBA3 tumor cells. After palpable tumors had formed animals were intratumorally injected with 2x10⁷ p.f.u. heat inactivated virus, WT VSV, VSV-IL-4 or VSV-TK. Virus treatment was repeated after 3 days. Tumor volumes at day 21 post-implantation (7 days after the last virus treatment) are shown. Results are presented as a mean ± S.E.M. (n=5). Comparable results were obtained in three independent sets of experiments.
Figure 4C. Induction of CTL response against B16(F10) tumor in animals receiving VSV-TK/GCV treatment. C57B1/6 tumor bearing mice were injected intratumorally with wild type or recombinant VSVs. A second injection was administered 3 days later. Ten days after the first virus injection, spleen cells were isolated and cocultured with B16(F10) cells. Spleen cells were incubated at the indicated effector to target ratios with ⁵¹Cr labeled B16(F10) target cells. CTL activity was determined by ⁵¹Cr release.

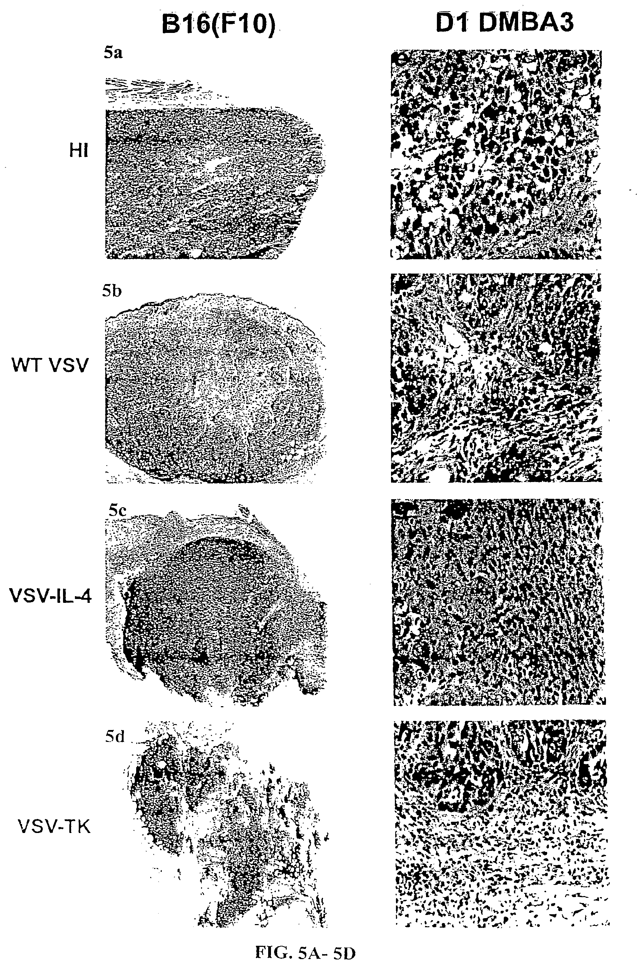

Figures 5A-5D illustrate the histopathological analysis of tumors. Tumors from C57B1/6 and Balb/c mice were removed 7 days after receiving intratumoral injections of either Figure 5A heat inactivated (HI)-VSV, Figure 5B WT VSV, Figure 5C VSV-IL-4, or Figure 5D VSV-TK. The left panel indicates large areas of cell death in B16(F10) tumors from WT VSV treated tumors, which are more pronounced in tumors treated with VSV-TK and VSV-IL-4. The right panel emphasizes increased infiltration of eosinophils in D1 DMBA3 tumors injected with VSV-IL-4.

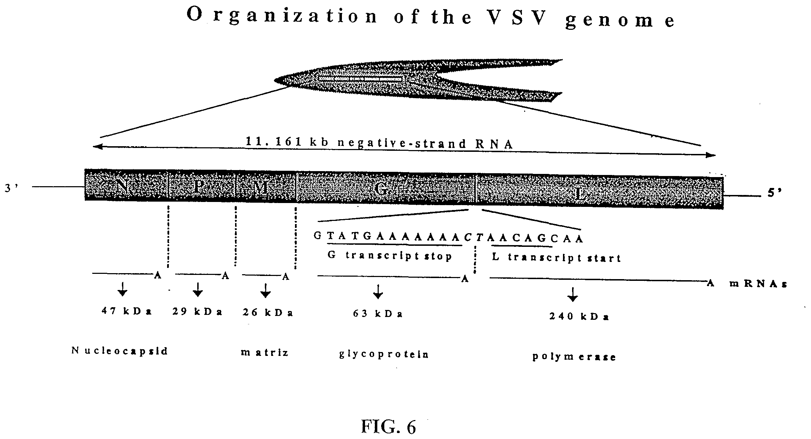

Figure 6 illustrates the genomic organization of the VSV genome.

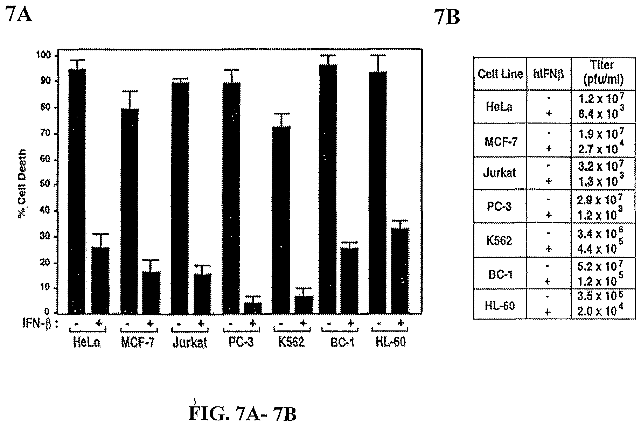

Figures 7A-7B demonstrate that several human cancer cell lines are permissive to VSV replication and lysis. Figure 7A demonstrates that MCF-7, BC-1, Jurkat, HL60, K562, PC-3 and HeLa cells were treated with or without 1000 U/ml hIFN-β for 18 hours and subsequently infected with VSV. 48 hours post infection, viability was assessed by Trypan blue exclusion analysis. Figure 7B. Supernatants from cells treated as in Figure 7A were analyzed for viral yield by standard plaque assay.

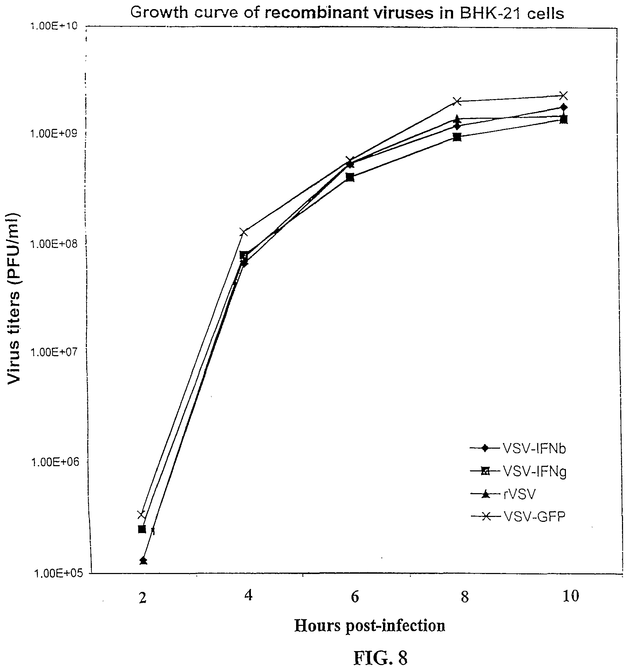

Figure 8 shows the growth curve of recombinant viruses expressing interferon in BHK-21 cells.

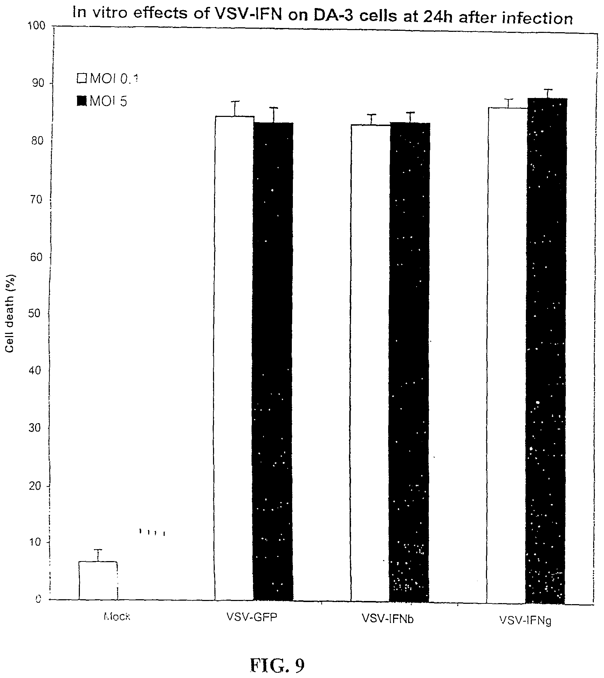

Figure 9 shows the *in vitro* effects of VSV-INF on DA-3 cells at 24 hours after infection.

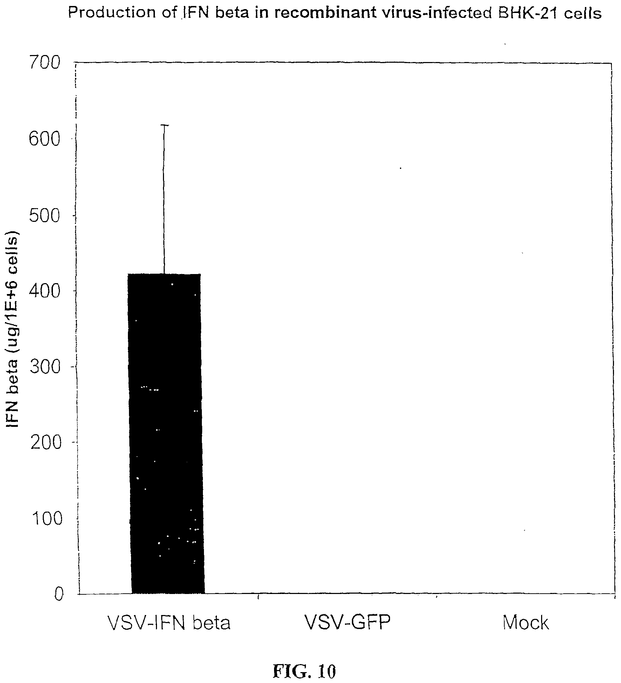

Figure 10 shows the production of INF-beta in recombinant virus-infected BHK-21 cells.

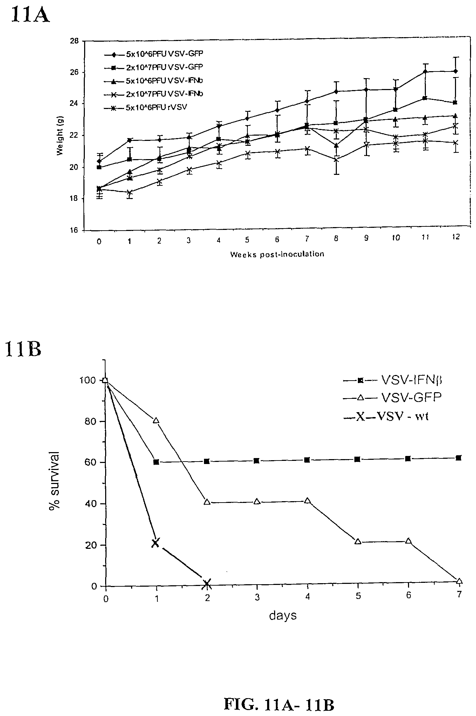

Figures 11A-11B show the effects of viral inoculation on weights and survivals. Fig. 11A shows the average weight of mice following virus inoculation. BALB/c mice (n=5 per group) were inoculated intravenously with VSV-IPNβ, VSV-GFP, or rVSV at 5x10⁶ or 2x10⁷ p.f.u. per mouse, and weights of mice were measured every week. Error bars show 0.5 x standard deviation. Fig. 11B shows the survival rate of mice following virus inoculation. BALB/c mice (n= 5 per group) were inoculated intravenously with 1x10⁸ p.f.u. of VSV-IFNβ, VSV-GFP, or wild-type VSV, and the mortality of mice was monitored daily.

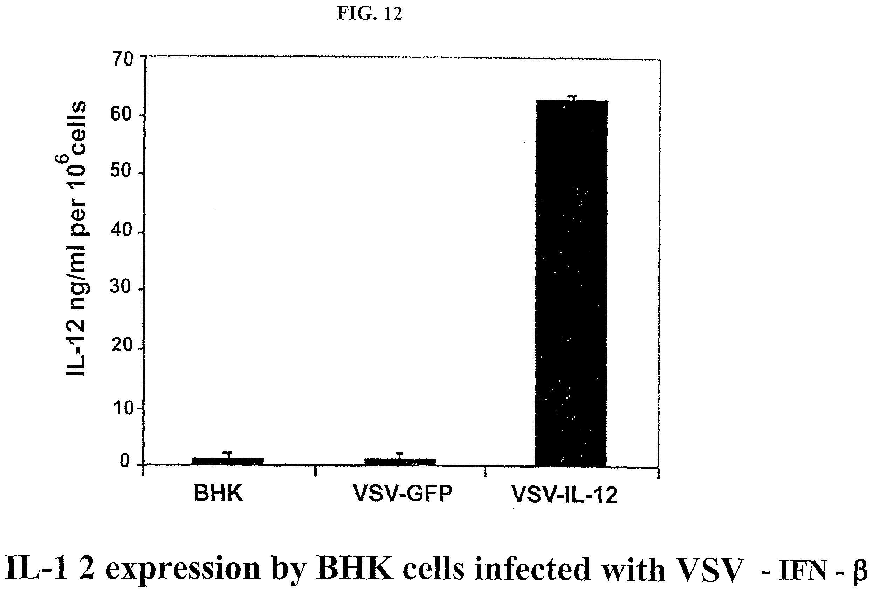

Figure 12 shows IL-12 expression by BHK cells infected with VSV-IL-12.

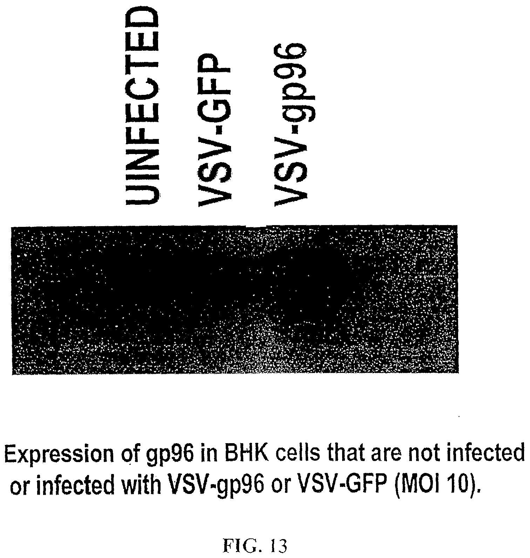

Figure 13 shows expression of gp96 in BHK cells that are not infected or infected with VSV-gp96 or VSV GFP (m.o.i. 10).

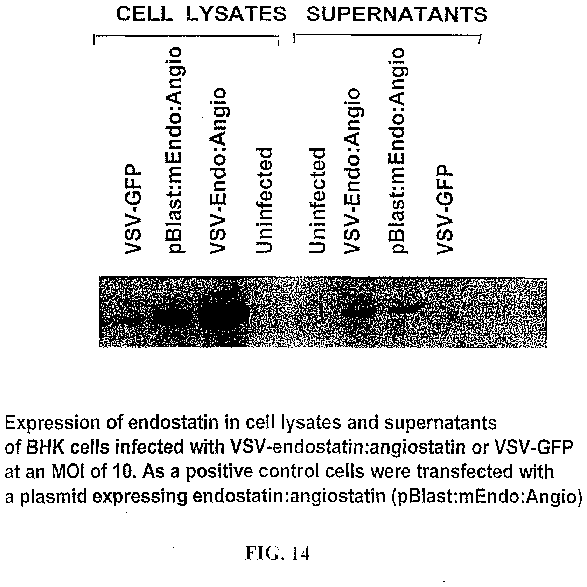

Figure 14 shows expression of endostatin in cell lysates and supernatants of BHK cells infected with VSV-endostatin:angiostatin or VSV-GFP at an m.o.i. of 10. As a positive control, cells were transfected with a plasmid expressing endostatin:angiostatin (pBlast:mEndo:Angio).

### DETAILED DESCRIPTION OF THE INVENTION

Vesicular stomatitis virus (VSV) is a negative-stranded virus, comprising only 5 genes, that preferentially replicates in immortalized and malignant cells, eventually inducing apoptosis. A schematic illustration of the VSV viral genome is shown in Figure 6. The ability of VSV to reproduce in tumor or malignant cells has been reported to occur, in part, to a defective interferon (IFN) system. Since the IFN system is functional in normal cells, efficient replication of VSV, which is an IFN-sensitive virus, is prevented. Based on *in vitro* and *in vivo* observations, it has been demonstrated that VSV effectively replicates in and lyses infected cancer cells, while leaving normal cells relatively unaffected. Stodj et al., *supra;* Fernandez et al. (2002, J. Virol. 76:895-904); Balachandran et al. (2001, J. Virol, 75:3474-9; Balachandran and Barber *supra.*

The use of VSV as an oncolytic agent has several advantages over other virus delivery systems presently used in tumor therapy such as adenoviruses and retroviruses. Foremost, VSV has no known transforming abilities. VSV is not gene-attenuated, which affects replication and therefore oncolytic anti-tumor activity. The envelope glycoprotein (G) of VSV is highly tropic for a number of cell-types and should be effective at targeting a variety of tissues *in vivo.* VSV appears to be able to replicate in a wide variety of tumorigenic cells and not, for example, only in cells defective in selective tumor suppressor genes such as p53. VSV is able to potently exert its oncolytic activity in tumors harboring defects in the Ras, Myc and p53 pathways, cellular aberrations that occur in over 90% of all tumors. VSV can be modified through genetic engineering to comprise immunomodulatory and/or suicide cassettes designed to increase the anti-tumor activity of the VSV.

Results of experiments disclosed herein demonstrate that a VSV vector comprising nucleic acid encoding a cytokine or suicide cassette exhibits greater oncolytic activity against tumor cells than a wild-type VSV vector alone. Results from experiments disclosed herein demonstrate that a VSV vector comprising nucleic acid encoding TK exhibits oncolytic activity against systemic and sub-cutaneous tumors and stimulates anti-tumor T-cell response. Data also demonstrate that VSV-IL4 or VSV-TK induce apoptosis, *in vivo,* of highly aggressive melanoma cells when an animal is infected at an m.o.i. of 1 or less. The data also demonstrate that VSV-TK and VSV-IL4 exhibit oncolytic activity superior to VSV alone in examples disclosed herein.

VSV has also been successfully used to express IFN-beta (Fig 10) or IFN-gamma. VSV-IFN can be grown in tumor cells since the IFN response is defective in these cells. Therefore, VSV-IFN still replicates in tumor cells to destroy them. During replication in the tumor cells, VSV makes high levels of IFN, which is secreted to surrounding cells. IFN is a powerful immunostimulatory molecule (these cytokines can activate dendritic cells and NK cells) and they also have tumor suppressive properties. Thus, the synthesis of IFN from VSV-IFN infected cells may induce additional anti-tumor affects and enhance the oncolytic activity of VSV. Normal cells surrounding the tumor should be activated (have their anti-viral state induced) by the VSV-synthesized IFN and become additionally protected against inadvertent VSV infection. In effect VSV-IFN should exert more potent anti-tumor activity than VSV alone and should be safer for normal, that is, non-tumor cells. The data disclosed herein indicate that VSV-IFN kills cancerous cells very efficiently, and normal cells are considerable more protected. Thus, VSV expressing IFN-beta is specific against cancer cells, more attenuated in normal cells, and therefore, safer. Results from experiments described herein demonstrate that a VSV vector comprising nucleic acid encoding IFN-beta or IFN-gamma replicates in cancerous cells and kills them. The data also demonstrate that VSV-IFN-beta and VSV-IFN-gamma exhibit oncolytic activity superior to VSV alone.

Data disclosed herein indicate that VSV vectors can be utilized to express high levels of biologically active recombinant proteins. Essentially, following virus infection, cellular transcription and translation is prevented, and cytoplasmic resources are focused on unbridled expression of the virus genes and any accompanying heterologous nucleic acid, even potentially toxic cellular or viral genes. Data disclosed herein demonstrate that VSV can be modified to deliver genes such as suicide and immunomodulatory cassettes that can greatly increase oncolytic activity, such as for example, killing of tumor cells. In addition, VSV possesses high target specificity and proficient transfection efficacy.

### General Techniques

The practice of the present invention will employ, unless otherwise indicated, conventional techniques of molecular biology (including recombinant techniques), microbiology, cell biology, biochemistry and immunology, which are within the scope of those of skill in the art. Such techniques are explained fully in the literature, such as, "Molecular Cloning: A Laboratory Manual", second edition (Sambrook et al., 1989); "Oligonucleotide Synthesis" (M.J. Gait, ed., 1984); "Animal Cell Culture" (R.I. Freshney, ed., 1987); "Methods in Enzymology" (Academic Press, Inc.); "Handbook of Experimental Immunology" (D.M. Weir & C.C. Blackwell, eds.); "Gene Transfer Vectors for Mammalian Cells" (J.M. Miller & M.P. Calos, eds., 1987); "Current Protocols in Molecular Biology" (F.M. Ausubel et al., eds., 1987); "PCR: The Polymerase Chain Reaction", (Mullis et al., eds., 1994); and "Current Protocols in Immunology" (J.E. Coligan et al., eds., 1991).

For general information related to vesicular stomatitis virus, see, "Fundamental Virology", second edition, 1991, ed. B. N. Fields, Raven Press, New York, pages 489-503; and "Fields Virology", third edition, 1995, ed. B.N. Fields, vol. 1, pages 1121-1159.

"VSV" as used herein refers to any strain of VSV or mutant forms of VSV, such as those described in WO 01/19380. A VSV construct of this invention may be in any of several forms, including, but not limited to, genomic RNA, mRNA, cDNA, part or all of the VSV RNA encapsulated in the nucleocapsid core, VSV complexed with compounds such as PEG and VSV conjugated to a nonviral protein. VSV vectors of the invention encompasses replication-competent and replication-defective VSV vectors, such as, VSV vectors lacking G glycoprotein.

As used herein, the terms "malignant", "malignant cells", "tumor", "tumor cells", "cancer" and "cancer cells", (used interchangeably) refer to cells which exhibit relatively autonomous growth, so that they exhibit an aberrant growth phenotype characterized by a significant loss of control of cell proliferation. The term "tumors" includes metastatic as well as non-metastatic tumors.

As used herein "oncolytic activity" refers to inhibition or suppression of tumor and/or malignant and/or cancerous cell growth; regression of tumor and/or malignant and/or cancerous cell growth; cell death of tumor and/or malignant and/or cancerous cells or prevention of the occurrence of additional tumor and/or malignant and/or cancerous cells. As used herein, "inhibiting or suppressing tumor growth" refers to reducing the rate of growth of a tumor, halting tumor growth completely, causing a regression in the size of an existing tumor, eradicating an existing tumor and/or preventing the occurrence of additional tumors upon administration of the VSV comprising compositions, or methods of the present invention. "Suppressing" tumor growth indicates a growth state that is curtailed when compared to growth without contact with a VSV of the present invention. Tumor cell growth can be assessed by any means known in the art, including, but not limited to, measuring tumor size, determining whether tumor cells are proliferating using a ³H-thymidine incorporation assay, or counting tumor cells. "Suppressing" tumor and/or malignant and/or cancerous cell growth means any or all of the following states: slowing, delaying, and stopping tumor growth, as well as tumor shrinkage. "Delaying development" of tumor and/or malignant and/or cancerous cells means to defer, hinder, slow, retard, stabilize, and/or postpone development of the disease. This delay can be of varying lengths of time, depending on the history of the disease and/or individual being treated.

The term "cytokine" as used herein includes any cytokine capable of stimulating an immune response in an individual. Such cytokines include, but are not limited to, interleukins, including but not limited to interleukin-2, interleukin-4, interleukin-6, interleukin-12; interferons, including but not limited to, interferon-alpha, interferon-beta, interferon-gamma, interferon-omega and interferon-epsilon; granulocyte-macrophage colony stimulating factors, and tumor necrosis factor. An "immunomodulatory" protein is one that can stimulate the immune system and includes, but is not limited to cytokines and chemokines.

The term "suicide cassette" or "suicide gene" (interchangeable herein) refer to genes that assist in killing tumor cells and include but are not limited to thymidine kinase and cytosine deaminase.

As used herein, the term "vector" refers to a polynucleotide construct designed for transduction/transfection of one or more cell types. VSV vectors may be, for example, "cloning vectors" which are designed for isolation, propagation and replication of inserted nucleotides, "expression vectors" which are designed for expression of a nucleotide sequence in a host cell, or a "viral vector" which is designed to result in the production of a recombinant virus or virus-like particle, or "shuttle vectors", which comprise the attributes of more than one type of vector. The present invention encompasses VSV vectors that comprise nucleic acid encoding cytokines, including but not limited to those cytokines described herein; chemokines, such as for example, Mip; co-stimulatory proteins, such as for example, B7-1 and B7-2; angiostatin; endostatin; and heat shock proteins, such as for example gp96.

The terms "polynucleotide" and "nucleic acid", used interchangeably herein, refer to a polymeric form of nucleotides of any length, either ribonucleotides or deoxyribonucleotides. These terms include a single-, double-or triple-stranded DNA, genomic DNA, cDNA, genomic RNA, mRNA, DNA-RNA hybrid, or a polymer comprising purine and pyrimidine bases, or other natural, chemically, biochemically modified, non-natural or derivatized nucleotide bases. The backbone of the polynucleotide can comprise sugars and phosphate groups (as may typically be found in RNA or DNA), or modified or substituted sugar or phosphate groups. Alternatively, the backbone of the polynucleotide can comprise a polymer of synthetic subunits such as phosphoramidates and thus can be a oligodeoxynucleoside phosphoramidate (P-NH2) or a mixed phosphoramidate- phosphodiester oligomer. Peyrottes et al. (1996) Nucleic Acids Res. 24: 1841-8; Chaturvedi et al. (1996) Nucleic Acids Res. 24: 2318-23; Schultz et al. (1996) Nucleic Acids Res. 24: 2966-73. A phosphorothioate linkage can be used in place of a phosphodiester linkage. Braun et al. (1988) J. Immunol. 141: 2084-9; Latimer et al. (1995) Molec. Immunol. 32: 1057-1064. In addition, a double-stranded polynucleotide can be obtained from the single stranded polynucleotide product of chemical synthesis either by synthesizing the complementary strand and annealing the strands under appropriate conditions, or by synthesizing the complementary strand *de novo* using a DNA polymerase with an appropriate primer. Reference to a polynucleotide sequence (such as referring to a SEQ ID NO) also includes the complement sequence.

The following are non-limiting examples of polynucleotides: a gene or gene fragment, exons, introns, genomic RNA, mRNA, tRNA, rRNA, ribozymes, cDNA, recombinant polynucleotides, branched polynucleotides, plasmids, vectors, isolated DNA of any sequence, isolated RNA of any sequence, nucleic acid probes, and primers. A polynucleotide may comprise modified nucleotides, such as methylated nucleotides and nucleotide analogs, uracyl, other sugars and linking groups such as fluororibose and thioate, and nucleotide branches. The sequence of nucleotides may be interrupted by non-nucleotide components. A polynucleotide may be further modified after polymerization, such as by conjugation with a labeling component. Other types of modifications included in this definition are caps, substitution of one or more of the naturally occurring nucleotides with an analog, and introduction of means for attaching the polynucleotide to proteins, metal ions, labeling components, other polynucleotides, or a solid support.

"Under transcriptional control" is a term well understood in the art and indicates that transcription of a polynucleotide sequence depends on its being operably (operatively) linked to an element which contributes to the initiation of, or promotes, transcription. "Operably linked" refers to a juxtaposition wherein the elements are in an arrangement allowing them to function.

In the context of VSV, a "heterologous polynucleotide" or "heterologous gene" or "transgene" is any polynucleotide or gene that is not present in wild-type VSV.

In the context of VSV, a "heterologous" promoter is one which is not associated with or derived from VSV.

A "host cell" includes an individual cell or cell culture which can be or has been a recipient of a VSV vector(s) of this invention. Host cells include progeny of a single host cell, and the progeny may not necessarily be completely identical (in morphology or in total DNA complement) to the original parent cell due to natural, accidental, or deliberate mutation and/or change. A host cell includes cells transfected, transformed or infected *in vivo* or *in vitro* with a VSV vector of this invention.

"Replication" and "propagation" are used interchangeably and refer to the ability of an VSV vector of the invention to reproduce or proliferate. These terms are well understood in the art. For purposes of this invention, replication involves production of VSV proteins and is generally directed to reproduction of VSV. Replication can be measured using assays standard in the art. "Replication" and "propagation" include any activity directly or indirectly involved in the process of virus manufacture, including, but not limited to, viral gene expression; production of viral proteins, nucleic acids or other components; packaging of viral components into complete viruses; and cell lysis.

An "individual" is a vertebrate, preferably a mammal, more preferably a human. Mammals include, but are not limited to, farm animals, sport animals, rodents, primates, e.g. humans, and pets.

An "effective amount" is an amount sufficient to effect beneficial or desired results, including clinical results. An effective amount can be administered in one or more administrations. For purposes of this invention, an effective amount of a VSV vector is an amount that is sufficient to palliate, ameliorate, stabilize, reverse, slow or delay the progression of the disease state.

"Expression" includes transcription and/or translation.

As used herein, the term "comprising" and its cognates are used in their inclusive sense; that is, equivalent to the term "including" and its corresponding cognates.

"A," "an" and "the" include plural references unless the context clearly dictates otherwise.

### VSV

### VSV sequences and constructs

VSV, a member of the *Rhabdoviridae* family, is a negative-stranded virus that replicates in the cytoplasm of infected cells, does not undergo genetic recombination or reassortment, has no known transforming potential and does not integrate any part of it genome into the host. VSV comprises an about 11 kilobase genome that encodes for five proteins referred to as the nucleocapsid (N), polymerase proteins (L) and (P), surface glycoprotein (G) and a peripheral matrix protein (M). The genome is tightly encased in nucleocapsid (N) protein and also comprises the polymerase proteins (L) and (P). Following infection of the cell, the polymerase proteins initiate the transcription of five subgenomic viral mRNAs, from the negative-sense genome, that encode the viral proteins. The polymerase proteins are also responsible for the replication of the full-length viral genomes that are packaged into progeny virions. The matrix (M) protein binds to the RNA genome/nucleocapsid core (RNP) and also to the glycosylated (G) protein, which extends from the outer surface in an array of spike like projections and is responsible for binding to cell surface receptors and initiating the infectious process.

Following attachment of VSV through the (G) protein to receptor(s) on the host surface, the virus penetrates the host and uncoats to release the RNP particles. The polymerase proteins, which are carried in with the virus, bind to the 3' end of the genome and sequentially synthesize the individual mRNAs encoding N, P, M, G, and L, followed by negative-sense progeny genomes. Newly synthesized N, P and L proteins associate in the cytoplasm and form RNP cores which bind to regions of the plasma membrane rich in both M and G proteins. Viral particles form and budding or release of progeny virus ensues.

A schematic illustration of the VSV genome is shown in Figure 6. A table of various VSV strains is shown in "Fundamental Virology", second edition, *supra,* at page 490. WO 01/19380 and U.S. Patent No. 6,168,943 disclose that strains of VSV include Indiana, New Jersey, Piry, Colorado, Coccal, Chandipura and San Juan. The complete nucleotide and deduced protein sequence of a VSV genome is known and is available as Genbank VSVCG, accession number JO2428; NCBI Seq ID 335873; and is published in Rose and Schubert, 1987, in The Viruses: The Rhabdoviruses, Plenum Press, NY. pp. 129-166. A complete sequence of a VSV strain is shown in U.S. Pat. No. 6,168,943. VSV New Jersey strain is available from the American Type Culture Collection (ATCC) and has ATCC accession number VR-159. VSV Indiana strain is available from the ATCC and has ATCC accession number VR-1421.

The present invention encompasses the use of any strain of VSV, including mutants of VSV disclosed in WO 01/19380. The present invention encompasses any form of VSV, including, but not limited to genomic RNA, mRNA, cDNA, and part or all of VSV RNA encapsulated in the nucleocapsid core. The present invention encompasses VSV in the form of a VSV vector construct as well as VSV in the form of viral particles. The present invention also encompasses nucleic acid encoding specific VSV vectors disclosed herein. As discussed herein, VSV vectors of the present invention encompass replication-competent as well as replication-defective VSV vectors.

Accordingly, the present invention provides recombinant vesicular stomatitis virus (VSV) vectors comprising nucleic acid encoding a cytokine, wherein said recombinant VSV vector exhibits greater oncolytic activity against a tumor cell than a wild-type VSV vector when contacted with the tumor cell. In some examples, the cytokine is an interferon, such as interferon-beta or interferon-gamma. In other examples, the cytokine is an interleukin such as for example, IL-4 or IL-12. The present invention encompasses VSV vectors comprising nucleic acid encoding more than one biologically active protein, such as for example, a VSV vector comprising nucleic acid encoding two cytokines, such as for example, an interferon and an interleukin; two interferons; or two interleukins. In one example, a VSV vector comprises nucleic acid encoding interferon-beta and interleukin-12. A VSV vector may comprise nucleic acid encoding a heat shock protein, such as gp96 and a cytokine, such as an interferon. In other examples, the VSV vector is replication-competent. In additional examples, the VSV vector is replication-defective. In yet other examples, the VSV vector lacks a protein function essential for replication, such as G-protein function or M and/or N protein function. The VSV vector may lack several protein functions essential for replication. In other examples, the tumor cell is a melanoma tumor cell, mammary tumor cell, prostate tumor cell, cervical tumor cell, hematological-associated tumor cell or a cell harboring a defect in a tumor suppressor pathway. The present invention also provides a replication-defective VSV vector comprising nucleic acid encoding interferon, wherein said recombinant VSV vector exhibits greater oncolytic activity against a tumor cell than a wild-type VSV vector when contacted with the tumor cell. In some examples, the interferon is interferon-beta or interferon-gamma. In other examples, the VSV vector lacks G-protein. In further examples, the tumor cell includes a melanoma tumor cell, mammary tumor cell, prostate tumor cell, cervical tumor cell, hematological-associated tumor cell or a cell harboring a defect in a tumor suppressor pathway. In further examples, an animal comprises the tumor cell and in other examples, the animal is a mammal, such as a human. The present invention also provides viral particles comprising a VSV vector of the present invention, such as a VSV vector comprising nucleic acid encoding a cytokine or suicide gene. The present invention also comprises isolated nucleic acid encoding a recombinant VSV vector of the present invention as well as host cells comprising a recombinant VSV vector of the present invention.

VSV is sensitive to the antiviral actions of the interferons (IFN). In studies with mice rendered defective in type I IFN signaling, the animals become susceptible to lethal infection by VSV. Data indicate that a functional IFN system is required to induce antiviral genes responsible for inhibiting the viral replication. One key anti-viral gene that is induced by IFN is referred to as the RNA-dependent protein kinase. (PKR), a 68 kDa serine/threonine protein kinase, that has been shown to be critical for protection against VSV infection. Down regulation of PKR protein or activity occurs in a broad spectrum of human malignancies. Therefore, tumor cells with reduced PKR activity are predicted to be more susceptible to VSV infection than their normal counterparts. WO 01/19380. Methods for measuring the activity of PKR in cells/cell lines are known in the art. Table 1 shows that interferon protects primary but not transformed cells from infection with rVSV. B16(F10) (murine melanoma), DA-3 (murine breast cancer) and HMVEC (human microvascular endothelial cells; normal cells) pretreated with IFN for 24 h were infected with WT VSV, VSV-IL-4, VSV-TK or VSV-GFP at an m.o.i. of 0.1 pfu for 18 h. Supernatants from infected cells were used to determine viral titers in plaque assays.

**Table 1. Viral titers in interferon treated transformed and primary cells**

| Cell line | Virus | PFU/ml |
|---|---|---|
| B16(F10) | WT VSV | 7.0x10⁵ |
| | VSV-TK | 1.0x10⁵ |
| | VSV-IL-4 | 6.2x10⁴ |
| | VSV-GFP | 4.0x10⁵ |
| | | |
| DA-3 | WT VSV | 2.9x10⁷ |
| | VSV-TK | 3.1x10⁷ |
| | VSV-IL-4 | 2.7x10⁷ |
| | VSV-GFP | 3.0x10⁷ |
| | | |
| HMVEC | WT VSV | <100 |
| | VSV-TK | <100 |
| | VSV-IL-4 | <100 |
| | VSV-GFP | <100 |

VSV replicates preferentially in malignant cells. This is primarily due to host defense mechanisms that normally contain VSV infection being damaged in cancerous cells, thus allowing the virus to propagate. The virus will destroy the malignant cells by mechanisms involving virus-induced apoptosis. Tumors grown in mice can be destroyed following intratumoral or intravenous inoculation of VSV. Table 2 provides a list of cell lines and VSV ability to replicate in these cell lines.

**Table 2**

| Cell line | Cell or tissue type | VSU infection |
|---|---|---|
| BHK | hamster kidney | + |
| HMVEC | human normal | - |
| B16 (F10) | melanoma | + |
| DA-3 | breast | + |
| MCF-7 | transformed hu breast | + |
| BC-1 | hu hematological malignancy | + |
| Jurkat | hu hematological malignancy | + |
| HL60 | hu hematological malignancy | + |
| K562 | hu hematological malignancy | + |
| PC-3 | hu transformed prostate | + |
| Hela | hu cervical tumor | + |

| | | |
|---|---|---|
| wherein "hu" refers to of human origin | | |

Recombinant VSV vectors that contain suicide cassettes and/or immunomodulatory genes are shown to enhance apoptotic or antitumor immune activity. Recombinant VSV vectors have been produced that contain nucleic acid encoding the IL-4 or IL-12 gene and that express large quantities of the IL-4 or IL-12 cytokine following infection of a cell. IL-4 and IL-12 are responsible for regulating T and B-cell responses. Without being bound by theory, rVSV-IL4 or VSV-IL-12 expressing constructs should target cancer cells in the body and replicate while producing amounts of localized IL-4 or IL-12, which may stimulate cytotoxic T-cell and/or antibody responses to the tumor. This may have an amplified antitumor effect and help eradicate the malignancy. Other immunomodulatory proteins that have been inserted into VSV constructs include the interferons, chemokines, endostatin, angiostatin and heat shock protein gp96.

Recombinant VSV that contains the suicide cassette TK gene has been constructed that expresses large quantities of thymidine kinase following VSV infection of the cell. Expression of the herpes simplex virus thymidine kinase (HSV- TK) in tumor cells allows the conversion of prodrugs such as gancyclovir (GCV) and acyclovir (ACV) into their monophosphate forms which are further phosphorylated by cellular kinases into their di- and triphosphate forms. The triphosphate metabolites then get incorporated into DNA and cause cell death by inhibiting mammalian DNA polymerases. Neighboring tumor cells that do not express this gene are also killed in the presence of GCV, the phenomenon known as "bystander killing". This effect is mediated by cellular connexins and gap junctions that allow the transfer of toxic metabolites into neighboring cells. As demonstrated herein VSV vectors comprising nucleic acid encoding TK demonstrates greater killing potential to a VSV vector alone. Additional VSV constructs that comprises nucleic acid encoding other suicide genes, such as cytosine deaminase, which renders cells capable of metabolizing 5-fluorocytosine (5-FC) to the chemotherapeutic agent 5-fluorouracil (5-FU), increases cell killing and bystander effect have been produced. As will be appreciated by the skilled artisan, other suicide genes may be employed. The addition of a suicide gene to a VSV vector may improve the safety of VSV therapy for immunocompromised individuals. A VSV vector comprising nucleic acid encoding cytosine deaminase fused to uracil phosphoribosyltransferase was constructed. This VSV vector exhibited functional expression of the cytosine deaminase activity.

VSV vector constructs comprising nucleic acid encoding interferon, and in particular interferon-beta and interferon-gamma, have been produced. As shown in Figure 10, high levels of functional INF-beta are produced by cells comprising a VSV vector construct comprising nucleic acid encoding INF-beta. As shown in Figure 9, VSV-IFN-beta and VSV-IFN-gamma increase cell death of DA-3 cells at 24 hours after VSV infection.

Additionally, recombinant VSVs efficiently produces large amounts of difficult to make/toxic/rare proteins. Thus, such viruses could be useful in making large amounts of Il-4, IL-12, IL-2 and other cytokines or other toxic or hard to make proteins. Accordingly, the invention includes VSV vectors encoding nucleic acid encoding angio and endostatin, heat shock and immune co-stimulatory molecules have been prepared. VSV vectors and VSV viral particles can be generated to make any protein of choice, in large amounts and constitutes a eukaryotic version of the successful baculovirus/insect cell expression system. Advantages of the VSV system include high level of expression and authentic (eukaryotic) processing, unlike in insect cells.

Accordingly, the present invention provides methods for making a recombinant VSV vector of the present invention comprising growing a cell comprising said VSV vector under conditions whereby VSV is produced; and optionally isolating said VSV. In some examples, the VSV vector is replication defective and the host cells comprising the VSV protein function essential for VSV replication such that said VSV vector is capable of replication in said host cell. In some examples, the VSV vector comprises nucleic acid encoding a cytokine, such as an interferon or interleukin; a suicide gene, such as thymidine kinase or cytosine deaminase or other biological protein, such as a heat shock protein, such as for example, gp96, and endostatin and angiostatin.

### Host cells, compositions and kits comprising VSV

The present invention also provides host cells comprising (i.e., transformed, transfected or infected with) the VSV vectors or particles described herein. Both prokaryotic and eukaryotic host cells, including insect cells, can be used as long as sequences requisite for maintenance in that host, such as appropriate replication origin(s), are present. For convenience, selectable markers are also provided. Host systems are known in the art and need not be described in detail herein. Prokaryotic host cells include bacterial cells, for example, *E. coli, B. subtilis,* and mycobacteria. Among eukaryotic host cells are yeast, insect, avian, plant, C. *elegans* (or nematode) and mammalian host cells. Examples of fungi (including yeast) host cells are S. *cerevisiae, Kluyveromyces lactis (K. lactis),* species of *Candida* including C. *albicans* and C. *glabrata, Aspergillus nidulans, Schizosaccharomyces pombe (S. pombe), Pichia pastoris,* and *Yarrowia lipolytica.* Examples of mammalian cells are COS cells, mouse L cells, LNCaP cells, Chinese hamster ovary (CHO) cells, human embryonic kidney (HEK) cells, and African green monkey cells. *Xenopus laevis* oocytes, or other cells of amphibian origin, may also be used.

The present invention also includes compositions, including pharmaceutical compositions, containing the VSV vectors described herein. Such compositions are useful for administration *in vivo,* for example, when measuring the degree of transduction and/or effectiveness of oncolytic activity toward a malignant cell. Compositions can comprise a VSV vector(s) of the invention and a suitable solvent, such as a physiologically acceptable buffer. These are well known in the art. In other embodiments, these compositions further comprise a pharmaceutically acceptable excipient. These compositions, which can comprise an effective amount of a VSV vector of the invention in a pharmaceutically acceptable excipient, are suitable for systemic or local administration to individuals in unit dosage forms, sterile parenteral solutions or suspensions, sterile non-parenteral solutions or oral solutions or suspensions, oil in water or water in oil emulsions and the like. Formulations for parenteral and nonparenteral drug delivery are known in the art and are set forth in Remington's Pharmaceutical Sciences, 19th Edition, Mack Publishing (1995). Compositions also include lyophilized and/or reconstituted forms of the VSV vectors (including those packaged as a virus) of the invention.

The present invention also encompasses kits containing VSV vector(s) of this invention. These kits can be used for example for producing proteins for screening, assays and biological uses, such as treating a tumor. Procedures using these kits can be performed by clinical laboratories, experimental laboratories, medical practitioners, or private individuals.

The kits of the invention comprise a VSV vector described herein in suitable packaging. The kit may optionally provide additional components that are useful in the procedure, including, but not limited to, buffers, developing reagents, labels, reacting surfaces, means for detection, control samples, instructions, and interpretive information. The kit may include instructions for administration of a VSV vector.

### Methods of producing recombinant VSV

The study of VSV and related negative strand viruses has been limited by the inability to perform direct genetic manipulation of the virus using recombinant DNA technology. The difficulty in generating VSV from DNA is that neither the full-length genomic nor antigenomic RNAs are infectious. The minimal infectious unit is the genomic RNA tightly bound to 1,250 subunits of the nucleocapsid (N) protein (Thomas et al., 1985, J. Virol. 54:598-607) and smaller amounts of the two virally encoded polymerase subunits, L and P. To reconstitute infectious virus from the viral RNA, it is necessary first to assemble the N protein-RNA complex that serves as the template for transcription and replication by the VSV polymerase. Although smaller negative-strand RNA segments of the influenza virus genome can be packaged into nucleocapsids in vitro, and then rescued in influenza infected cells (Enami et al., 1990, Proc. Natl. Acad. Sci. USA 87:3802-3805; Luytjes et al., 1989, Cell 59:1107-1113), systems for packaging the much larger eukaryotic genomic RNAs *in vitro* are not yet available.

Systems for replication and transcription of DNA-derived minigenomes or small defective RNAs from Rhabdoviruses (Conzelmann and Schnell, 1994, J. Virol. 68:713-719; Pattnaik et al., 1992, Cell 69:1011-1120) have been described. In these systems, RNAs are assembled into nucleocapsids within cells that express the viral N protein and polymerase proteins. These systems do not allow genetic manipulation of the full-length genome of infectious viruses. U.S. Patent No. 6,168,943 discloses methods for the preparation of infectious recombinant vesiculovirus capable of replication in an animal into which the recombinant vesiculovirus is introduced. For example, U.S. Patent No. 6,168,943 describes that vesiculoviruses are produced by providing in an appropriate host cell: (a) DNA that can be transcribed to yield (encode) vesiculovirus antigenomic (+) RNA (complementary to the vesiculovirus genome), (b) a recombinant source of vesiculovirus N protein, (c) a recombinant source of vesiculovirus P protein, and (d) a recombinant source of vesiculovirus L protein; under conditions such that the DNA is transcribed to produce the antigenomic RNA, and a vesiculovirus is produced that contains genomic RNA complementary to the antigenomic RNA produced from the DNA.

Alternatively, after purification of genomic RNA, VSV mRNA can be synthesized *in vitro,* and cDNA prepared by standard methods, followed by insertion into cloning vectors (see, e.g., Rose and Gallione, 1981, J. Virol. 39(2):519-528). VSV or portions of VSV can be prepared using oligonucleotide synthesis (if the sequence is known) or recombinant methods (such as PCR and/or restriction enzymes). Polynucleotides used for making VSV vectors of this invention may be obtained using standard methods in the art, such as chemical synthesis, recombinant methods and/or obtained from biological sources. Individual cDNA clones of VSV RNA can be joined by use of small DNA fragments covering the gene junctions, generated by use of reverse transcription and polymerase chain reaction (RT-PCR) (Mullis and Faloona, 1987, Meth. Enzymol. 155:335-350) from VSV genomic RNA (see Section 6, infra). The ability to recover fully infectious virus from a plasmid cDNA copy of the VSV genome has allowed genetic manipulation of this virus to become feasible.

In an example disclosed herein, a cDNA clone representing the entire 11,161 nucleotides of VSV has been generated and unique Xho I/Nhe I sites were added to facilitate entry of a heterologous gene, e.g. for example, HSV-TK. Transcription of the cDNA is dependent on T7 RNA polymerase. Vaccinia vTF7-3 was used to infect baby hamster kidney cells (BHK-21), to provide a source of polymerase. Subsequently, VSV cDNA was transfected into the same cells together with three other plasmids that express the VSV N, P and L proteins. These latter three proteins facilitate the assembly of nascent VSV antigenomic RNA into nucleocapsids and initiate the VSV infectious cycle. After 24 hours, host cells were lysed, clarified and residual vaccinia removed by filtration through a 0.2 um filter onto fresh BHK cells. Only recombinant VSVs are produced by this method since no wild-type VSV can be generated (Rose et al., 1995, P.N.A.S. USA).

VSV may be genetically modified in order to alter it properties for use *in vivo.* Methods for the genetic modification of VSV are well established within the art. For example, a reverse genetic system has been established for VSV (Roberts et al., Virology, 1998, 247:1-6) allowing for modifications of the genetic properties of the VSV. Standard techniques well known to one of skill in the art may be used to genetically modify VSV and introduce desired genes within the VSV genome to produce recombinant VSVs (see for example, Sambrooke et al., 1989, A Laboratory Manual, New York: Cold Spring Harbor Laboratory Press. For insertion of nucleotide sequences into VSV vectors, for example nucleotide sequences encoding a cytokine, or for VSV gene sequences inserted into vectors, such as for the production helper cell lines, specific initiation signals are required for efficient translation of inserted protein coding sequences. These signals include the ATG initiation codon and adjacent sequences. In cases where an entire VSV gene, such as G-protein including its own initiation codon and adjacent sequences are inserted into the appropriate vectors, no additional translational control signals may be needed. However, in cases where only a portion of the gene sequence is inserted, exogenous translational control signals, including the ATG initiation codon, must be provided. The initiation codon must furthermore be in phase with the reading frame of the protein coding sequences to ensure translation of the entire insert. These exogenous translational control signals and initiation codons can be of a variety of origins, both natural and synthetic.

Following infection of a host cell, recombinant VSV shuts down host cell protein synthesis and expresses not only its own five gene products, but also heterologous proteins encoded within its genome. Successful expression of heterologous nucleic acid from VSV recombinants requires only the addition of the heterologous nucleic acid sequence into the full-length cDNA along with the minimal conserved sequence found at each VSV gene junction. This sequence consists of the polyadenylation/transcription stop signal (3' AUACU₇) followed by an intergenic dinucleotide (GA or CA) and a transcription start sequence (3' UUGUCNNUAG) complementary to the 5' ends of all VSV mRNAs. Ball et al. 1999, J. Virol. 73:4705-4712; Lawson et al. 1995, P.N.A.S. USA 92:4477-4481; Whelan et al. 1995, P.N.A.S. USA 92:8388-8392. Additionally, restriction sites, preferably unique, (e.g., in a polylinker) are introduced into the VSV cDNA, for example in intergenic regions, to facilitate insertion of heterologous nucleic acid, such as nucleic acid encoding an interleukin or interferon. In other examples, the VSV cDNA is constructed so as to have a promoter operatively linked thereto. The promoter should be capable of initiating transcription of the cDNA in an animal or insect cell in which it is desired to produce the recombinant VSV vector. Promoters which may be used include, but are not limited to, the SV40 early promoter region (Bernoist and Chambon, 1981, Nature 290:304-310), the promoter contained in the 3' long terminal repeat of Rous sarcoma virus (Yamamoto, et al., 1980, Cell 22:787-797), the herpes thymidine kinase promoter (Wagner et al., 1981, Proc. Natl. Acad. Sci. U.S.A. 78:1441-1445), the regulatory sequences of the metallothionein gene (Brinster et al., 1982, Nature 296:39-42); heat shock promoters (e.g., hsp70 for use in Drosophila S2 cells); the ADC (alcohol dehydrogenase) promoter, PGK (phosphoglycerol kinase) promoter, alkaline phosphatase promoter, and the following animal transcriptional control regions, which exhibit tissue specificity and have been utilized in transgenic animals: elastase I gene control region which is active in pancreatic acinar cells (Swift et al., 1984, Cell 38:639-646; Ornitz et al., 1986, Cold Spring Harbor Symp. Quant. Biol. 50:399-409; MacDonald, 1987, Hepatology 7:425-515); insulin gene control region which is active in pancreatic beta cells (Hanahan, 1985, Nature 315:115-122), immunoglobulin gene control region which is active in lymphoid cells (Grosschedl et al., 1984, Cell 38:647-658; Adames et al., 1985, Nature 318:533-538; Alexander et al., 1987, Mol. Cell. Biol. 7:1436-1444), mouse mammary tumor virus control region which is active in testicular, breast, lymphoid and mast cells (Leder et al., 1986, Cell 45:485-495), albumin gene control region which is active in liver (Pinkert et al., 1987, Genes and Devel. 1:268-276), alpha-fetoprotein gene control region which is active in liver (Krumlauf et al., 1985, Mol. Cell. Biol. 5:1639-1648; Hammer et al., 1987, Science 235:53-58; alpha 1-antitrypsin gene control region which is active in the liver (Kelsey et al., 1987, Genes and Devel. 1:161-171), beta-globin gene control region which is active in myeloid cells (Mogram et al., 1985, Nature 315:338-340; Kollias et al., 1986, Cell 46:89-94; myelin basic protein gene control region which is active in oligodendrocyte cells in the brain (Readhead et al., 1987, Cell 48:703-712); and myosin light chain-2 gene control region which is active in skeletal muscle (Sani, 1985, Nature 314:283-286). Preferably, the promoter is an RNA polymerase promoter, preferably a bacteriophage or viral or insect RNA polymerase promoter, including but not limited to the promoters for T7 RNA polymerase, SP6 RNA polymerase, and T3 RNA polymerase. If an RNA polymerase promoter is used in which the RNA polymerase is not endogenously produced by the host cell in which it is desired to produce the recombinant VSV, a recombinant source of the RNA polymerase must also be provided in the host cell. Such RNA polymerase are known in the art.

The VSV cDNA can be operably linked to a promoter before or after insertion of nucleic acid encoding a heterologous protein, such as a mammalian protein including a cytokine or a suicide gene. In some examples, a transcriptional terminator is situated downstream of the VSV cDNA. In other examples, a DNA sequence that can be transcribed to produce a ribozyme sequence is situated at the immediate 3' end of the VSV cDNA, prior to the transcriptional termination signal, so that upon transcription a self-cleaving ribozyme sequence is produced at the 3' end of the antigenomic RNA, which ribozyme sequence will autolytically cleave (after a U) this fusion transcript to release the exact 3' end of the VSV antigenomic (+) RNA. Any ribozyme sequence known in the art may be used, as long as the correct sequence is recognized and cleaved. (It is noted that hammerhead ribozyme is probably not suitable for use.)

VSV vectors of the present invention comprise one or more heterologous nucleic acid sequence(s) encoding a mammalian protein, such as for example, a cytokine, suicide gene or heat shock protein gp96, or a reporter gene, such as for example, green fluorescent protein. Examples of cytokines include, but are not limited to interferons (IFN), including IFN-beta, IFN-gamma, INF-alpha, INF-omega, and INF-epsilon; tumor necrosis factor (TNF), lymphotoxin, interleukins (IL), including but not limited to IL-2, IL-4, and IL-12 and granulocyte-macrophage colony-stimulating factor (GM-CSF). A VSV vector may comprise nucleic acid encoding two cytokines, such as for example, two interleukins, two interferons or an interleukin and an interferon.

The sequences for most of the genes encoding IFN as they occur in nature are published and many have been deposited with the American Type Culture Collection (ATCC) (Rockville, Md.). A VSV vector of the present invention can encode any form of IFN. In some examples, the nucleic acid encodes a human form of IFN. This includes human IFN, IFN-alpha, IFN-beta, IFN-gamma, IFN-omega, and INF-epsilon. Human INF-alpha sequences are described in Weber et al. (1987, EMBO, J. 6:591-598); human INF-beta sequences are described in U.S. Pat. No. 5,908,626 and Fiers et al. (1982) Philos. Trans. R. Soc. Lond., B, Biol. Sci. 299:29-38) and has been deposited with GenBank under Accession No. M25460; human INF-gamma is available from the ATCC and has ATCC accession numbers 39047 and 39046 and a particular form of recombinant human IFN-gamma is commercially available (rhIFN-gamma-lb, Actimmune.RTM., Genentech, Inc. South San Francisco, Calif.); and human IFN-epsilon sequence are disclosed in U.S. Pat. No. 6,329,175. The human IL-2 gene has been cloned and sequenced and can be obtained as, for example, a 0.68 kB BamHI-HinDIII fragment from pBC12/HIV/IL-2 (available from the American Type Culture Collection ("ATCC") under Accession No. 67618). U.S. Pat. No. 5,951,973 discloses the sequence for mouse and human IL-4. Interleukin-12 (IL-12), originally called natural killer cell stimulatory factor, is a heterodimeric cytokine described, for example, in M. Kobayashi et al, J. Exp. Med., 170:827 (1989). The expression and isolation of IL-12 protein in recombinant host cells is described in detail in International Patent Application WO90/05147, published May 17, 1990 (also European patent application No. 441,900). The DNA and amino acid sequences of the 30 kd and 40 kd subunits of the heterodimeric human IL-12 are provided in the above recited international application. Research quantities of recombinant human and murine IL-12 are also available from Genetics Institute, Inc., Cambridge, Massachusetts. Further, the sequences of human GM-CSF, human TNF and human lymphotoxin are known and are available. The sequence of human GM-CSF is known (Wong et al. (1985) Science 228:810-815) and has been deposited with GenBank under Accession No. M10663. The sequence of human TNF has been described (Wang et al. (1985) Science 228:149-154) and is deposited with GenBank under Accession No. M10988. The sequence of human lymphotoxin (TNF-b) has also been published (Iris et al. (1993) Nature Genet. 3:137-145) and is deposited with GenBank under Accession No. Z15026.

A VSV vector or viral particle of the present invention can comprise nucleic acid encoding a suicide gene, such as thymidine kinase (TK), such as herpes simplex TK or cytosine deaminase (CD), such as for example *E.coli* CD. The HSV-TK gene has been previously mapped, cloned and sequenced, and is readily available (EMBL HEHSVLTK, Accession X03764, EMBL HEHS07, Accession V00466). The HSV-tk gene can be obtained from natural sources, such as from the viral genome of Herpes simplex virus type I (HSV-1) or from the Herpes simplex virus type II (HSV-2) genome. The varicella zoster virus (VZV) genome also includes a specific thymidine kinase gene (VZV-tk) which has been cloned, sequenced and characterized (Mori et al. (1988) Intervirology 29:301-310, (1986) J. Gen. Virol. 67:1759-1816). Thus, the VZV-tk gene can be obtained from the VZV genome. The E. coli cytosine deaminase gene has also been cloned and sequenced (Danielson et al. (1992) Mol. Microbiol. 6:1335-1344, Austin et al. (1993) Mol. Pharmacol. 43:380-387, Dong et al. (1996) Human Gene Therapy 7:713-720), and the gene sequence has been deposited with GenBank under Accession No. S56903. The *E. coli* cytosine deaminase gene can therefore also be obtained from a number of natural or synthetic sources known to those skilled in the art. Alternatively, cytokine or suicide gene oligonucleotides can be synthetically derived, using a combination of solid phase direct oligonucleotide synthesis chemistry and enzymatic ligation methods which are conventional in the art. Synthetic sequences can be prepared using commercially available oligonucleotide synthesis devices such as those devices available from Applied Biosystems, Inc. (Foster City, Calif.).

The present invention encompasses expression systems comprising a VSV vector comprising one or more heterologous nucleotide sequence(s), such as, a nucleotide sequence encoding a cytokine, such as for example, interferon, or two cytokines, or a suicide gene, such as for example, TK, inserted within a region of the VSV essential for replication, such as the G glycoprotein region, or other region essential for replication, such that the VSV lacks the essential function and is replication-defective. The VSV vector may have a mutation, such as a a point mutation or deletion of part or all, of any region of the VSV genome, including the G, M, N, L or P region. If the mutation is in a region essential for replication, the VSV will be grown in a helper cell line that provides the essential region function. The VSV may also comprise a mutation, such as for example, a point mutation or deletion of part or all of a nucleotide sequence essential for replication, and optionally, with the heterologous nucleotide sequence inserted in the site of the deleted nucleotide sequence. The heterologous nucleotide sequence may be operably linked to a transcriptional regulatory sequence. Following infection of a target malignant or tumor cell, progeny viruses will lack essential protein function and cannot disseminate to infect surrounding tissue. In additional embodiments, the VSV vector is mutated in nucleic acid, such as by point mutation, substitution or addtion of nucleic acid, or deletion of part or all, of nucleic acid encoding other VSV protein function such as, M protein and/or N protein function. VSV may be targeted to a desired site *in vitro* to increase viral efficiency. For example, modification of VSV G protein (or other VSV proteins) to produce fusion proteins that target specific sites may be used to enhance VSV efficiency *in vivo.* Such fusion proteins may comprise, for example, but not limited to single chain Fv fragments that have specificity for tumor antigens. (Lorimer et al., P.N.A.S. U.S.A., 1996. 93:14815-20).

A VSV vector lacking a gene(s) essential for viral replication can be grown in an appropriate complementary cell line. Accordingly, the present invention provides recombinant helper cell lines or helper cells that provide a VSV protein function essential for replication of a replication-deficient VSV construct. In some examples, the protein function is G-protein function. For example, a VSV vector comprising nucleic acid encoding a cytokine and lacking G-protein function can be grown in a cell line, i.e., a helper cell line, for example, a mammalian cells line such as CHO cell line, permissive for VSV replication, wherein said cell line expresses an appropriate G-protein function, such that said VSV is capable of replicating in the cell line. These complementing or helper cell lines are capable of allowing a replication-defective VSV to replicate and express one or more foreign genes or fragments thereof encoded by the heterologous nucleotide sequence. In some embodiments, the VSV vector lacks a protein function essential for replication, such as for example, G-protein function and the host cell line comprises nucleic acid encoding the protein function essential for replication, such as for example, VSV G-protein function. Complementing cell lines can provide VSV viral function through, for example, co-infection with a helper virus, or by integration or otherwise maintaining in stable form part or all of a viral genome encoding a particular viral function. In other examples, additional VSV non-essential proteins can be deleted or heterologous nucleotide sequences inserted into nucleotide regions encoding non-essential VSV, such as for example, the M and N proteins. The heterologous nucleotide sequence can be inserted into a region non-essential for replication wherein the VSV is replication-competent. Heterologous nucleotide sequences can be inserted in non-essential regions of the VSV genome, without necessitating the use of a helper cell line for growth of the VSV vector.

The recombinant VSV of the invention are produced for example, by providing in an appropriate host cell VSV cDNA wherein said cDNA comprises nucleotide sequence encoding a heterologous protein, such as for example, a cytokine, including interleukin or interferon, or a suicide gene. The nucleic acid encoding a heterologous protein can be inserted in a region non-essential for replication, or a region essential for replication, in which case the VSV is grown in the presence of an appropriate helper cell line. In some examples, the production of recombinant VSV vector is *in vitro,* in cell culture, in cells permissive for growth of the VSV. Standard recombinant techniques can be used to construct expression vectors containing DNA encoding VSV proteins. Expression of such proteins may be controlled by any promoter/enhancer element known in the art. Promoters which may be used to control expression of VSV proteins can be constitutive or inducible.

The host cell used for recombinant VSV production can be any cell in which VSV grows, e.g., mammalian cells and some insect (e.g., Drosophila) cells. Primary cells lacking a functional INF system, or in other examples, immortilized or tumor cell lines can be used. A vast number of cell lines commonly known in the art are available for use. By way of example, such cell lines include but are not limited to BHK (baby hamster kidney) cells, CHO (Chinese hamster ovary) cells, HeLA (human) cells, mouse L cells, Vero (monkey) cells, ESK-4, PK-15, EMSK cells, MDCK (Madin-Darby canine kidney) cells, MDBK (Madin-Darby bovine kidney) cells, 293 (human) cells, and Hep-2 cells. Such cell lines are publicly available for example, from the ATCC and other culture depositories.

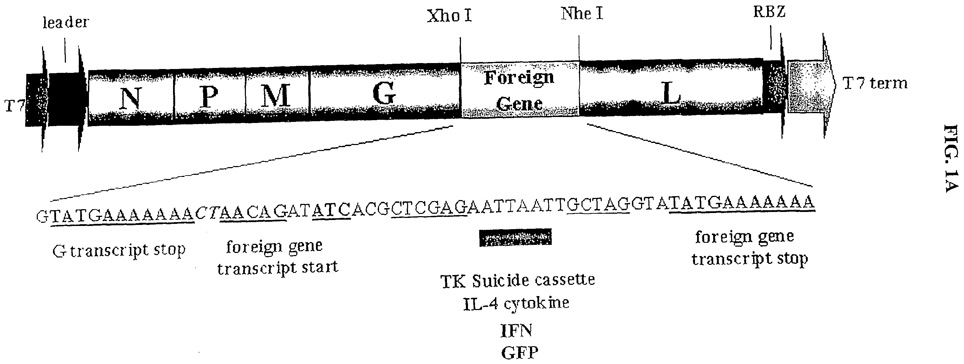

In examples disclosed herein, the plasmid, pVSV-XN2 was constructed as shown in Fig. 1A. The genes encoding HSV-TK, mouse IL-4, INF-beta or INF-gamma or GFP were cloned into the pVSV-XN2, between the VSV G and L genes. Recombinant VSV (rVSV) produced by cell lines can be isolated using for example, an affinity matrix. Method of isolating VSV by affinity matrix are described in for example, WO 01/19380. Briefly, methods for isolating a rVSV from comprising adding the VSV to an affinity matrix, to produce bound VSV, washing the bound VSV, and eluting the VSV from the affinity matrix. The present invention encompasses a modified VSV that comprises a non-naturally occurring fusion protein on the outer surface of the virus. The non-native protein may be a fusion protein comprising an affinity tag and a viral envelope protein or it may be derived from a producer cell. Producer cell lines may be engineered to express one or more affinity tags on their plasma membranes which would be acquired by the virus as it buds through the membrane. One example of an affinity tag is the use of Histidine residues which bind to immobilized nickel columns. Affinity tags also include antibodies. Other protocols for affinity purification may be used as known within the art, for example, but not limited to, batch processing, a solution of virus and affinity matrix, pelleting the VSV-bound matrix by centrifugation, and isolating the virus. Alternatively, VSV can be collected and purified as described in U.S. Patent No. 6,168,943. Briefly, VSV is collected from culture supernatants, and the supernatants clarified to remove cellular debris. One method of isolating and concentrating the virus is by passage of the supernatant through a tangential flow membrane concentration. The harvest can be further reduced in volume by pelleting through a glycerol cushion and by concentration on a sucrose step gradient.

### Methods of using recombinant VSV vectors of the invention

The subject VSV vectors and viral particles can be used for a wide variety of purposes, which will vary with the desired or intended result. Accordingly, the present invention includes methods using the VSV vectors described herein.

The invention provides methods for producing oncolytic activity in a tumor cell and/or malignant cell and/or cancerous cell comprising contacting the cell, including, for example, a tumor cell or a malignant cell in metastatic disease, with a VSV vector of the invention, wherein said VSV vector exhibits greater oncolytic activity against the cell than a wild-type VSV vector. In some examples, the contacting is effected by administration, such as for example, intravenous injection to an individual comprising said cell. In other examples, the contacting is effected by administration, such as by intratumoral injection to an individual comprising said cell. For these methods, the VSV vector may or may not be used in conjunction with other treatment modalities for producing oncolytic activity, such as, for example, tumor suppression, including but not limited to chemotherapeutic agents known in the art, radiation and/or antibodies. The invention also provides compositions comprising a VSV vector comprising nucleic acid encoding a cytokine or a suicide gene wherein said VSV vector is present in the composition in an amount effective to produce oncolytic activity when said composition is administered to the tumor and/or malignant cells. In some examples, the composition further comprises a pharmaceutical excipient. In other examples, the composition is administered intratumorally or intravenously to an individual comprising the tumor cells.

Accordingly, the present invention provides methods for producing oncolytic activity in a tumor cell, comprising the step of contacting the cell with a recombinant VSV vector comprising nucleic acid encoding a cytokine, wherein said VSV vector exhibits greater oncolytic activity against the tumor cell than a wild-type VSV vector. In some examples of the methods, the VSV vector is replication-defective. In other examples, the VSV vector lacks G-protein function. In yet further examples, the cytokine is an interferon, such as for example, interferon-beta or interferon-gamma; or a cytokine, such as for example, an interleukin, such as interleukin-4 or interleukin-12. In additional examples, the tumor cell includes a melanoma tumor cell, mammary tumor cell, prostate tumor cell, cervical tumor cell, hematological-associated tumor cell or cell harboring defects in a tumor suppressor pathway. In yet further examples, said contacting is by intravenous injection to an individual comprising said tumor cell or by intratumoral injection to an individual comprising said tumor cell.

The present invention also provides methods for producing oncolytic activity in a tumor cell, comprising the step of contacting the tumor cell with a recombinant VSV vector comprising nucleic acid encoding a suicide gene wherein said VSV vector exhibits greater oncolytic activity against the tumor cell when administered along with a prodrug than a wild-type VSV vector. In some examples of the methods, the suicide gene encodes thymidine kinase (TK) and the prodrug is ganclyclovir or acyclovir. In other examples, the suicide gene encodes a cytosine deaminase and the prodrug is 5-fluorocytosine. In some examples of the methods, the VSV vector is replication-defective. In other examples, the VSV vector lacks G-protein function. In yet other examples of the methods, the tumor cell includes melanoma tumor cell, mammary tumor cell, prostate tumor cell, cervical tumor cell, hematological-associated tumor cell or cell harboring a defect in a tumor suppressor pathway. In other examples, the contacting is by intravenous injection to an individual comprising said tumor cell or by intratumoral injection to an individual comprising said tumor cell.

The invention also provides the use of the VSV vectors or compositions of the invention in the preparation of a medicament for treating a tumor. VSV was shown to induce cell death in transformed human cell lines including those derived from breast (MCF7), prostate (PC-3), or cervical tumors (HeLa), as well as a variety of cells derived from hematological-associated malignancies (HL 60, K562, Jurkat, BC-1). BC-1 is positive for human herpesvirus-8 (HHV-8), overexpresses Bcl-2 and is largely resistant to a wide variety of apoptotic stimuli and chemotherapeutic strategies. The results of additional studies indicated that VSV could induce apoptosis of cells specifically transformed with either Myc or activated Ras and transformed cells carrying Myc or activated Ras or lacking p53 or overexpressing Bcl-2 are susceptible to VSV replication and viral-induced apoptosis. Figures 7A-7B illustrate that several human cancer cell lines are permissive to VSV replication and lysis. Therefore, it is predicted that administration of a VSV vector of the present invention or a composition comprising a VSV vector of the present invention would produce oncolytic activity in a variety of malignant cells or tumor cells. Methods for screening cells or cell lines, including malignant cells lines, for susceptibility to infection with a VSV vector of the present invention, can be performed by methods disclosed in WO 01/19380. Briefly a method for identifying a tumor susceptible to treatment with a virus, comprises: (a) dividing a sample containing cells of the tumor into a first portion and a second portion; (b) treating portion with the VSV virus; and (c) determining whether the percentage of dead cells in the first portion is higher than in the second portion, wherein the tumor is susceptible to treatment with the VSV virus if the percentage of dead cells in the first portion is higher than in the second portion.

The present invention encompasses the use of VSV vector(s) or compositions of the invention in the preparation of a medicament for treating a tumor. Vectors or compositions of the present invention may be used for treating individuals with malignant cells and/or tumor cells susceptible to VSV infection, as described above. Also indicated are individuals who are considered to be at risk for developing tumor or malignant cells, such as those who have had previous disease comprising malignant cells or tumor cells or those who have had a family history of such tumor cells or malignant cells. Determination of suitability of administering VSV vector(s) of the invention will depend on assessable clinical parameters such as serological indications and histological examination of cell, tissue or tumor biopsies. Generally, a composition may comprise VSV vector(s) in a pharmaceutically acceptable excipient.

Accordingly, the vectors or compositions may be used in methods for suppressing tumor growth, comprising the step of contacting the tumor with a recombinant VSV vector comprising nucleic acid encoding a cytokine, wherein said VSV vector exhibits greater tumor suppression than a wild-type VSV vector. In some examples of the methods, the VSV vector is replication-defective. In other examples, the VSV vector lacks G-protein function. In yet further examples, the cytokine is an interferon, such as for example, interferon-beta or interferon-gamma; or a cytokine, such as for example, an interleukin, such as interleukin-4 or interleukin-12. The methods for suppressing tumor growth may comprise the step of contacting the tumor with a recombinant VSV vector comprising nucleic acid encoding a suicides gene wherein said VSV vector exhibits greater tumor suppression when administered along with a prodrug than a wild-type VSV vector. In some examples of the methods, the VSV vector is replication-defective. In other examples, the VSV vector lacks G-protein function. In yet further examples, the suicide gene is thymidine kinase and the prodrug is ganclyclovir or acyclovir. In other examples, the suicide gene encodes a cytosine deaminase and the prodrug is 5-fluorocytosine. In yet other examples of the methods, the tumor cell includes melanoma tumor cell, mammary tumor cell, prostate tumor cell, cervical tumor cell, hematological-associated tumor cell or cell harboring a defect in a tumor suppressor pathway.

*Ex vivo* treatment of cells or tissues using a VSV vector(s) in individuals with malignant cells and/or tumor cells and/or cancerous cells susceptible to VSV infection, as described above is envisaged. *Ex vivo* treatment of cells can be undertaken in an attempt to reduce or eliminate undesirable malignant or cancerous cells from a mixture of cells. Such cells include for example, bone marrow cells or peripheral blood stem cells. Accordingly, the VSV vector(s) may be used in a method for the *ex-vivo* treatment of cells whereby a cell population from an individual comprising undesirable cells or suspected of comprising undesirable cells is contacted with a VSV vector of the present invention, such as a VSV vector comprising nucleic acid encoding a cytokine or a suicide gene. After the contacting, the cell population maybe transplanted back into the individual. *Ex vivo* purging of cells using viruses is described in for example, WO 02/00233.

The VSV vectors, including VSV vectors comprising nucleic acid encoding one or more cytokine(s) such as for example, an IFN, including IFN-beta and IFN-gamma, may be used to infect tumor and/or malignant and/or cancerous cells, *in* *vitro.* The VSV vector replicates in the tumor cells, expressing the cytokine (immunomodulatory gene) to high levels. The cells are inoculated into an animal, (in some examples, back into the animal from which they were obtained) which makes an immune response to the infected, lysed tumor cells. The VSV expressed cytokines stimulate the host's immune response. Thus, an individual, such as a mammal, including a human, could be protected from subsequent tumor challenge, if exposed to the tumor and/or malignant and/or cancerous cells that have been contacted with a VSV vector of the present invention and subsequently lysed, thereby creating a "cancer vaccine" effect. The use of VSV vectors of the present invention as "cancer vaccines" can be tested in a non-human animal model by obtaining tumors grown in a mouse; contacting the tumors with a VSV vector of the present invention, such as a VSV vector comprising nucleic acid encoding a cytokine, such as for example, an IFN, IL-12 and/or heat shock protein, *in vitro.* Then different mice (same strain) that have the same tumor type growing in them are inoculated with the VSV-infected tumor cells. The VSV infected tumor cells lyse in the animal, attract the host's immune system and eradicate the established tumor (post-vaccine). In contrast, lysed tumor cells not exposed to virus are poor immunogens. The use of VSV virus infection attracts an immune response in the animal. In some examples, the VSV vector is replication-defective. In other examples, the VSV vector is replication-competent. Accordingly, the present invention provides compositions capable of eliciting an immune response in an individual comprising tumor cells infected with or lysed by a VSV vector of the present invention. Methods for eliciting an immune response to tumor cells in an individual may comprise administering a composition comprising tumor cells infected with or lysed by a VSV vector of the present invention to said individual. In some examples, the VSV vector comprises nucleic acid encoding one or more cytokines, such as an interferon or interleukin. In other examples, the VSV vector comprises nucleic acid encoding a immunomodulatory protein, such as a chemokine; or a heat shock protein, such as for example, gp96. Methods for protecting an individual against tumor challenge may comprise, contacting tumor cells derived from an individual with a VSV vector comprising nucleic acid encoding a cytokine, an immunomodulatory protein or a heat shock protein, such as gp96, under conditions suitable for lysing said tumor cells; and returning said lysed tumor cells to said individual.

Once a VSV vector comprising a nucleotide sequence encoding a cytokine or suicide gene has been obtained, the VSV vector, or VSV particles comprising the vector, can be administered to an individual in need. Such an individual can comprise malignant cells or tumor cells or can be at risk for developing malignant cells or tumor cells or development of metastatic disease. The VSV constructs of the present invention comprising a cytokine or suicide cassette can be used to treat local tumors or metastatic disease. A variety of cells and cells lines, including ovarian carcinoma cells, fibrosarcoma, lung carcinoma, melanoma, prostate carcinoma, lung carcinoma and leukaemia cells are sensitive to VSV infection. Therefore, such tumor cells and/or malignant cells derived therefrom may be particularly amenable to treatment with a VSV expressing a cytokine or a suicide gene. As disclosed herein, VSV expressing cytokines or suicide genes have been shown to exhibit greater oncolytic activity than wt VSV. It is expected that VSV will have oncolytic activity when administered locally to the tumor cells or malignant cells, that is intratumorally, as well as when administered distal to the tumor or malignant cell, such as *via* intravenous administration or by other routes.

To evaluate whether genetically engineered VSV carrying immunomodulatory or suicide genes, such as thymidine kinase, can be created and whether such viruses are more efficacious in tumor therapy than the wild type VSV, VSV vectors carrying the herpes virus thymidine kinase suicide cassette (TK) or the cytokine gene interleukin-4 (IL-4) were developed. It is known that the mechanism of TK action involves the TK protein phosphorylating the nontoxic pro-drug ganciclovir (GCV), which becomes incorporated into cellular DNA during replication leading to chain termination and cell death. The TK/GCV suicide approach has been reported to have additional benefits in that modified TK can be directly passed from the transduced cell to adjacent cells thereby increasing tumor killing, a phenomenon known as the "bystander effect". The activities of IL-4, in contrast, involve influencing the development of effector cells such as eosinophils and antigen presenting cells. IL-4 is also known to regulate T helper (Th) cell development into Th2 cells and assist in the stimulation of a humoral response (Asnagli et al. 2001, Curr. Opin. Immunol. 13: 242-7). High levels of IL-4 have been reported to be critical for the rejection of tumors in the initial phases of tumor development and implanted engineered IL-4 secreting cells as well as viral vectors transducing IL-4 have been shown to mediate the regression of a number of malignancies including melanoma, glioma and colon carcinoma (Benedetti et al. 2000, Nat. Med. 6:447-50; Giezeman-Smits, 2000, Cancer Res 60:2449-50; Nagai, et al. 2000, Breast Cancer 7: 181-6; Tepper et al. 1992, Science 257:548-51).

Results from experiments disclosed herein demonstrate that a VSV vector comprising nucleic acid encoding TK exhibits oncolytic activity against systemic and sub-cutaneous tumors and stimulates anti-tumor T-cell response. Data also demonstrate that VSV-IL4 or VSV-TK induce apoptosis, *in vivo,* of highly aggressive melanoma cells when an animal is infected at an m.o.i. of 1 or less. The data also demonstrate that VSV-TK and VSV-IL4 exhibit oncolytic activity superior to VSV alone in examples disclosed herein.

VSV vectors comprising nucleic acid encoding interferon, in particular, interferon-beta and interferon-gamma, were developed. Results from experiments described herein demonstrate that a VSV vector comprising nucleic acid encoding INF-beta or INF-gamma replicates in malignant cells and kills them. The data also demonstrate that VSV-INF-beta and INF-gamma exhibit oncolytic activity superior to VSV alone.

### Methods of administration

Many methods may be used to administer or introduce the VSV vectors or viral particles into individuals, including but not limited to, oral, intradermal, intramuscular, intraperitoneal, intravenous, intratumor, subcutaneous, and intranasal routes. The individual to which a VSV vector or viral particle is administered is a primate, or in other examples, a mammal, or in other examples, a human, but can also be a non-human mammal including but not limited to cows, horses, sheep, pigs, fowl, cats, dogs, hamsters, mice and rats. In the use of a VSV vector or viral particles, the individual can be any animal in which a VSV vector or virus is capable of growing and/or replicating. The present invention encompasses compositions comprising VSV vector or viral particles wherein said compositions can further comprise a pharmaceutically acceptable carrier. The amount of VSV vector(s) to be administered will depend on several factors, such as route of administration, the condition of the individual, the degree of aggressiveness of the malignancy, and the particular VSV vector employed, . Also, the VSV vector may be used in conjunction with other treatment modalities.

If administered as a VSV virus, from about 10² up to about 10⁷ p.f.u., in other examples, from about 10³ up to about 10⁶ p.f.u., and in other examples, from about 10⁴ up to about 10⁵ p.f.u. can be administered. If administered as a polynucleotide construct (i.e., not packaged as a virus), about 0.01 µg to about 100 µg of a VSV construct of the present invention can be administered, in other examples, 0.1 µg to about 500 µg, and in other examples, about 0.5 µg to about 200 µg can be administered. More than one VSV vector can be administered, either simultaneously or sequentially. Administrations are typically given periodically, while monitoring any response. Administration can be given, for example, intratumorally, intravenously or intraperitoneally.

Pharmaceutically acceptable carriers are well known in the art and include but are not limited to saline, buffered saline, dextrose, water, glycerol, sterile isotonic aqueous buffer, and combinations thereof. One example of such an acceptable carrier is a physiologically balanced culture medium containing one or more stabilizing agents such as stabilized, hydrolyzed proteins, lactose, etc. The carrier is preferably sterile. The formulation should suit the mode of administration.

The composition, if desired, can also contain minor amounts of wetting or emulsifying agents, or pH buffering agents. The composition can be a liquid solution, suspension, emulsion, tablet, pill, capsule, sustained release formulation, or powder. Oral formulation can include standard carriers such as pharmaceutical grades of mannitol, lactose, starch, magnesium stearate, sodium saccharine, cellulose, magnesium carbonate, etc.

Generally, the ingredients are supplied either separately or mixed together in unit dosage form, for example, as a dry lyophilized powder or water free concentrate in a hermetically sealed container such as an ampoule or sachette indicating the quantity of active agent. Where the composition is administered by injection, an ampoule of sterile diluent can be provided so that the ingredients may be mixed prior to administration.

In a specific embodiment, a lyophilized recombinant VSV of the invention is provided in a first container; a second container comprises diluent consisting of an aqueous solution of 50% glycerin, 0.25% phenol, and an antiseptic (e.g., 0.005% brilliant green).

The precise dose of VSV vector or viral particles to be employed in the formulation will also depend on the route of administration, and the nature of the patient, and should be decided according to the judgment of the practitioner and each patient's circumstances according to standard clinical techniques. The exact amount of VSV vector or virus utilized in a given preparation is not critical, provided that the minimum amount of virus necessary to produce oncolytic activity is given. A dosage range of as little as about 10 mg, up to amount a milligram or more, is contemplated.

Effective doses of the VSV vector or viral particle of the invention may also be extrapolated from dose-response curves derived from animal model test systems. A table of safety of recombinant viruses on BALB/c mice is shown below.

**Table 3**

| Table Safety of recombinant viruses on BALB/c mice | | | | |
|---|---|---|---|---|
| Inoculation | Virus | Amount of virus (PFU) | N. of dead mouse | Mortality (%) |
| I.V. | VSV-GFP | 1x10^6 | 0/5 | 0 |
| | | 5x10^6 | 0/5 | 0 |
| | | 2x10^7 | 0/5 | 0 |
| | | 5x10^7 | 0/5 | 0 |
| | | 1x10^8 | 5/5 | 100 |
| | VSV-IFNβ | 1x10^6 | 0/6 | 0 |
| | | 5x10^6 | 0/6 | 0 |
| | | 2x10^7 | 0/6 | 0 |
| | | 5x10^7 | 0/5 | 0 |
| | | 1x10^8 | 2/5 | 40 |
| | VSV-IFNγ | 1x10^6 | 0/6 | 0 |
| | | 5x10^6 | 0/6 | 0 |
| | | 2x10^7 | 0/6 | 0 |
| | | 5X10^7 | 4/5 | 80 |
| | rVSV | 1x10^6 | 0/5 | 0 |
| | | 5x10^6 | 0/5 | 0 |
| | VSV-IL12 | 2x10^7 | 0/2 | 0 |
| | | 5x10^7 | 2/2 | 100 |
| | | 1x10^8 | 4/4 | 100 |

The data show that the growth of VSV-IFN-beta is attenuated compared to VSV-GFP. EXAMPLES

### Example 1

### Materials and Methods

### Experimental Protocol

**Cell lines.** BHK-21 and B16(F10) melanoma cells were obtained from American Type Culture Condition (ATCC, Manassas, Virginia). BHK cells were grown in Dulbecco's Modified Essential Medium (DMEM) containing 10% fetal bovine serum (FBS, Hyclone Laboratories Inc, Logan, UT). B16(F10) cells were propagated in similar medium except that it contained low sodium bicarbonate (1.5 g/L). D1-DMBA3 breast tumor and DA-3 cells derived from the tumor were a gift from Dr. Diana Lopez (University of Miami, Miami, FL). Human primary cells (microvascular endothelial-HMVEC) were obtained from Clonetics Corp (San Diego, CA) and grown according to the manufacturer's specifications.

**Construction of recombinant viruses.** The IL-4, TK and GFP inserts were amplified from pGexp, pKO-TK and pLEGFP-C1 (Clontech Laboratories, Palo Alto, CA) plasmids respectively by PCR. For IL-4, the primers 5' GGCA*CTCGAG***ATGGGTCTCAACCCCCAGCTAGTTG** and 5' GCCG*TCTAGA***CTACGAGTAATCCATTTGCATGATGC** were used.

For the GFP gene, the primers used were 5' GGCA*CTCGAG***ATGGTGAGCAAGGGCGAGGAG** and 5' GCTTGAGC*TCTAGA*TCTGAGTCC**CTACTTGTACAGC**.

The TK gene was amplified using the following primers 5' CTTGTAGA*CTCGAGT***ATGGCTTCGTACCCCGGCCATCAG** 5' GTATTGTCTG*CTAGC*GTGTT**TCAGTTAGCCTCCCCCATC**.

The IL-4 and GFP PCR products were digested with *Xho*I and *Xba*I while the TK PCR product was digested with *Xho*I and *Nhe*I and ligated to pVSV-XN2 (a gift from Dr. John Rose, Yale University) that had been digested with *Xho*I and *Nhe*I (compatible with *Xba*I). The plasmid pVSV-XN2 contains the entire VSV genome and has unique *Xho*I and *Nhe*I sites flanked by VSV transcription start and stop signals. The procedure for recovering infectious recombinant VSV viruses was similar to that described previously (Lawson et al. 1995, P.N.A.S. USA 92:4477-81; Whelan et al., 1995, P.N.A.S. USA 92:8388-92).

**In vitro cell killing assay.** Murine B16, DA-3 and human HMVECs cells were seeded in 12 well plates at approximately 2x10⁵ cells/well. Cells were pretreated with interferon (human interferon-α-2a [Hoffman-La Roche, Nutley NJ] for HMVECs, mouse interferon-αβ [Sigma, St Louis, MI] for B16 and DA-3) at 1000 u/ml for 24 hours. The cells were then infected with wt VSV, VSV-TK, VSV-IL-4 or VSV-GFP at an m.o.i. of 0.1 for 18 hours, trypsinized and counted by Trypan Blue exclusion analysis.

**Enzyme-linked immunosorbent assay for IL-4 production.** Levels of IL-4 in the rVSV-IL-4 supernatants obtained 24 hours following infection of BHK cells with rVSV-IL-4 were determined by using an IL-4 ELISA kit obtained from Pharmingen (San Diego, CA) following the manufacturer's protocol.

**Thymidine kinase enzyme assay.** Phosphorylation of ganciclovir was used to measure functional levels of HSV-TK in extracts of VSV-TK infected cells as follows (Yamamoto et al. 1997, Cancer Gene Ther 4:91-6). After a 24 hour infection with wild type or recombinant viruses, BHK cells were washed twice with PBS and resuspended in a buffer containing 0.5% NP-40, 50 mM Tris HCl (pH 7.5), 20% glycerol, 5mM benzamidine, 2mM dithiothreotol and 0.5 mM PMSF. They were subjected to four cycles of freeze thawing followed by centrifugation of the lysates at 13,000 rpm for 5 min. at 4° C. The enzyme assay was carried out using 60 µg protein in a reaction mix containing 50 mM Tris HCl, 5 mM magnesium chloride, 5 mM ATP, 10 mM sodium fluoride and 2 mM dithiothreotol in a 37° C water bath. 20 µl aliquots were taken at 0, 30 and 60 minutes following addition of [³H] GCV (45 µM) and spotted on DE-81 (Whatman) paper. The filter papers were washed twice in 1 mM ammonium formate and extracted with 0.1M KCl / 0.1N HCl and counted in a scintillation counter.

**Tumor inoculation in mice.** Female C57B1/6 or Balb/c mice (8 week old) from Jackson laboratories were inoculated subcutaneously with 5x 10⁵ B16(F10) melanoma cells (left flank) or 1.5x10° Dl-DMBA3 breast tumor cells (mammary pad). Mice were divided into four groups of five each according to the type of virus administered - heat inactivated VSV, wild type VSV virus, VSV-TK or VSV-IL-4. After the development of palpable tumors the mice received 2x10⁷ pfu of the wild type or recombinant VSV viruses intratumorally followed by a second injection three days later. Mice that received VSV-TK were also administered GCV (100 mg/ kg body weight) one day following the initial injection, followed by daily injections for 10 days thereafter. Tumors were measured three times weekly. Mice were sacrificed once tumors reached greater than 1.8 cm in any diameter. Mean tumor volumes in the four groups were compared using one way ANOVA analysis. Histopathology was carried out as described previously (Balachandran et al. 2001, J. Virol. 75:3474-9).

**Cytotoxic assays.** Single cell spleen suspensions were cultured in 25 mm upright flasks at a 20:1 ratio with mitomycin C treated B16F10 cells for 3 days in the presence of 400 pg/ml IL-2 (Calbiochem, San Diego, CA) at 37°C in a humidified 5% CO₂ atmosphere. The cytotoxic activity of spleen cells was determined by performing a standard chromium release assay using 0.1 mCi of Na₂⁵¹CrO4 (Amersham, Arlington Heights, IL) at 37°C, 2 h. After 4 h incubation of effector and target cells, the supernatants were harvested using a SKATRON cell harvester and the amount of ⁵¹Cr release determined in a gamma counter (Beckman, Palo Alto, CA). The percent specific lysis was calculated by the following equation: experimental release CPM-spontaneous release CPM/ maximum release CPM- spontaneous release CPM X 100. Maximum release was the cpm obtained by incubating target cells with 2%SDS (Fisher), and spontaneous release was determined by incubation with growth medium alone. Spontaneous release of ⁵¹Cr was always less than 15% of the total release in these assays.

### Example 2

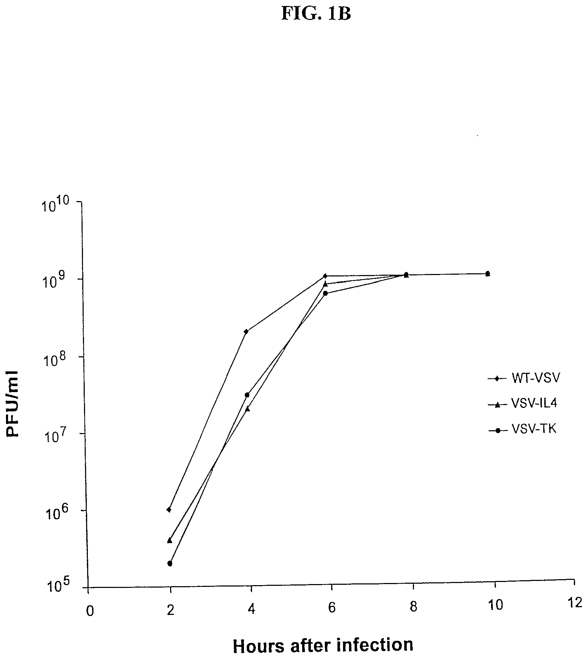

**Generation of rVSV expressing TK or IL-4.** To evaluate whether VSV could be generated to express potential anticancer genes, the HSV-TK, mouse IL-4 or, control green fluorescent protein (GFP) were cloned into the plasmid pVSV-XN2, as additional transcription units between the VSV G and L genes (Fig 1*a*). Recombinant VSVs expressing either TK, IL-4 or GFP from the modified transcription unit were recovered in cells expressing the full-length antigenomic VSV RNA containing the additional gene as well as the VSV nucleocapsid (N), phosphoprotein (P) and polymerase (L) proteins. Preliminary analysis indicated that viable recombinant viruses could be obtained in all cases and examination of virus production per cell, by one-step growth curve studies, indicated no aberrant variation in replication abilities compared to the wild-type VSV (Fig 1*b*). Indeed, all viruses grew to exceptionally high titers of approximately 10⁹ plaque forming units (pfu) per ml. We next determined whether the recovered VSVs expressed TK, IL-4, or GFP. Accordingly, BHK cells were infected with VSV-TK for 24 hours at a multiplicity of infection (m.o.i.) of 1 and infected cells were examined for TK protein expression by immunoblot analysis using an anti-TK monoclonal antibody. Results indicated that the TK protein was being synthesized to extremely high levels in cells infected with VSV-TK, while in contrast we were unable to detect any TK being expressed in cells infected with other types of VSV (Fig 2b lanes 1-3). Confirmation of TK expression was demonstrated using immunofluorescent microscopy of VSV-TK infected cells while BHK cells infected with VSV- green fluorescent protein (GFP) also expressed high levels of GFP as determined by fluorescent microscopy. To ascertain whether the TK was functional, GCV phosphorylation levels were measured in cells infected at an m.o.i. of 1 with VSV-TK or wild-type virus, 8 hours post-infection. The results indicated that TK phosphorylated GCV at high levels, on average approaching 200 pmoles/mg/min, which we estimated to be nearly 60-fold greater than in wild-type VSV infected cells (Fig 2*a*). The data disclosed herein thus indicate that VSV can be generated to express high levels of functional TK, without any adverse effects upon virus replication.

We next analyzed whether VSV could express functional IL-4. Since IL-4 is rapidly secreted from the cell following translation, preliminary immunoblot analysis of VSV-IL-4 infected cell extracts using an anti-murine IL-4 monoclonal antibody indicated very little IL-4 present in the cytoplasm of infected cells, as expected. However, capture enzyme-linked immunoabsorbant assay (ELISA) using a monoclonal antibody that binds functional IL-4 indicated that the cytokine was being secreted into the culture medium at very high levels from VSV-IL-4 infected cells (Fig. 2c). Indeed, VSV-IL-4 generated about 150 ng/ml of IL-4 per 10⁶ cells, over a hundred fold greater than in the same number of BHK cells transfected with IL-4 cDNA under control of the CMV promoter. Confirmation of IL-4 expression, was obtained by immunoprecipitating secreted IL-4 from the culture medium of ³⁵S labeled VSV-IL-4 infected cells using an anti-IL-4 monoclonal antibody (Fig. 2d). Thus, similar to our observations characterizing VSV-TK, we demonstrate that VSV can also be engineered to express high levels of the cytokine IL-4, which is biologically active.

### Example 3

**rVSV expressing TK or IL-4 retain oncolytic activity.** To evaluate whether the recombinant viruses expressing IL-4 or TK retained their ability to preferentially replicate in malignant cells, to eventually induce cell death, a number of transformed cells were examined in infection assays. An important objective was also to compare the effects of VSV and recombinant VSVs on normal cells. To start to appraise this, we selected human microvascular endothelial cells (HMVECs) since they would be most likely to be exposed to VSV infection after subcutaneous (s.c.) or intravenous (i.v.) administration in tumor therapy. HMVECs (10⁶) were therefore infected at an m.o.i of 0.1 for 18 hours with wild-type VSV or VSV-IL-4, VSV-TK or VSV-GFP. Cell viability was measured using Trypan Blue exclusion analysis and revealed that approximately 20-30% of the HMVECs underwent cell death, an effect that could be essentially eliminated following pre-treatment with interferon (IFN-α) [Fig. 3*a* and *d*]. In contrast, a similarly infected murine breast tumor cell-line (Sotomayor, et al. 1991, J. Immunol. 147:2816-23) (DA-3, derived from D1 DMBA-3 tumor) as well as a melanoma cell-line B16(F10) (Fidler et al., 1975, Cancer Res. 35, 218-24) underwent dramatic cytolysis (80-90%) following infection with either the wild-type virus, VSV-TK, VSV-IL-4 or VSV-GFP. VSV-TK induced potent cytolysis of cells even in the absence of GCV. Pretreatment of B16(F10) cells with IFN (1000 u/ml for 24 hours) reduced the amount of virus-mediated cell death observed following infection, regardless of the virus used. It remained plausible that the mechanisms of IFN production may be predominantly defective in B16(F10) cells in view of the fact that IFN-signaling to induce antiviral protection appears partially intact. However, subsequent analysis of viral production in IFN pre-treated B16(F10) cells revealed relatively high virus replication (10⁵/ml), suggesting incomplete protection and anti-viral activity (Table 1). In contrast, Trypan Blue exclusion analysis indicated that IFN did not afford any significant protection of breast tumor derived DA-3 cells, suggesting that IFN function may be defective at multiple levels in these cells (Fig. 3c and *ƒ*). These data were confirmed by determining that virus replication in IFN-treated DA-3 cells was exceedingly high (10⁷/ml) compared to control HMVECs in which virus production was almost completed ablated (Table 1). To evaluate the mechanisms of virus-mediated cell lysis, infected B16(F10) and DA-3 cells were evaluated for apoptotic activity 18 hours post-infection. The mechanisms of cytolysis invoked by recombinant VSV-IL-4 or TK as well as the wild-type virus involved the induction of apoptosis since levels of active caspase-8 and 9 was three-fold higher than in untreated cells. Thus, recombinant VSV expressing IL-4 or TK do not appear to lose their effectiveness at inducing programmed cell death in infected cells compared to wild-type VSV.

### Example 4

### rVSV expressing TK or IL-4 kill tumors in vivo.

To evaluate the oncolytic activity of the recombinant viruses, immunocompetent mice were sub-cutaneously (s.c.) implanted with 1 x 10⁶ cells of the syngeneic B16 melanoma (C57B1/6) or poorly immunogenic mammary tumor derived D1 DMBA (Balb/c), both aggressive tumors. Following the formation of palpable tumors, 2 x 10⁷ wild-type VSV or VSV-IL-4 or VSV-TK were introduced intratumorally (i.t.). As controls, an equivalent amount of heat-inactivated (HI) VSV was used. Ganciclovir was administered (100mg/kg body weight), intraperitoneally (i.p.), daily in animals receiving VSV-TK. Virus therapy was repeated once more, three days after the first injection and tumor growth monitored three times weekly. Resultant data demonstrated that wild-type VSV inhibited the growth of both the melanoma and breast tumors compared to tumors treated with control HI virus (Fig. 4*a* and *b*). However, in independent sets of experiments, more potent inhibition of tumor growth (both B16 and D-1 DMBA) was observed in animals treated with either VSV-IL-4 or VSV-TK (Fig. 4*a* and *b*). In some instances, complete regression of tumors was observed in animals implanted with either B16(F10) (3/5 mice) or D1 DMBA (2/5 mice), following treatment with VSV-TK. In contrast, a number of mice implanted with either tumor and infected with HI-VSV had to be sacrificed 4 days post-treatment because of the excessive tumor size. The differences in the tumor volume between the control group (HI-VSV) and animals treated with either VSV-TK or VSV-IL-4 was observed to be statistically significant, at (p<0.01) and (p<0.001) for animals implanted with B16(F10) or D1 DMBA, respectively. These data indicate that VSV expressing either IL-4 or TK exhibit potent oncolytic activity, superior to that of VSV alone.

To examine the effects of virus therapy, *in vivo,* tumors inoculated with the various viruses were excised and sections examined histologically following hematoxylin and eosin (H&E) staining. As expected, tumors infected with control HI-VSV exhibited very little morphological abnormalities that could be associated with virus induced oncolytic activity (<30% necrosis [Fig 5*a*]). However, a greater proportion of cell death, as exhibited by pyknotic nuclei, was observed in B16(F10) tumors treated with wild-type VSV (∼50%) or with VSV-IL-4 (75%) or VSV-TK (∼95%), indicative of an increase in oncolytic activity (Fig. *5b**-d*). Significant inflammatory infiltration was also evident in tumors treated with the recombinant viruses, especially in tumors treated with VSV-IL-4, which showed major infiltration of polymorphonuclear cells including neutrophils and eosinophils (Fig. 5 compare e to g). Analysis of viral replication in the brain, liver and tumors retrieved from mice implanted with B16(F10) or D1 DMBA cells and treated with wild-type or recombinant viruses (two were analyzed from each virus treated group) did not reveal evidence of infectious virus 7 days after the last virus treatment (one week after the primary inoculation). Thus, following i.t. inoculation, all VSV types appear to be rapidly cleared from the animals.

Although CD8+ T-lymphocytes have been reported to be important for the antitumor activity of IL-4, cellular immune responses have also been reported to play a role in the local and systemic antitumor activity of TK/GCV (Yamamoto et al. 1997, Cancer Gene Ther 4:91-6). Since IL-4 and TK/GCV treated tumors exhibited pronounced host cell infiltration, we examined whether lymphocytes generated by the animals exhibited specific cytotoxic activity to tumor associated antigens in the implanted syngeneic transformed cells. Accordingly, animals harboring B16(F10) derived tumors were intratumorally inoculated twice with wild-type VSV, HI VSV, VSV-IL-4 or VSV-TK. Seven days after inoculation, spleens were removed, mononuclear cells isolated and chromium release assays carried out using B16(F10) target cells. The results indicated that animals generated a robust CTL-response only against tumors receiving VSV-TK, and not with VSV-IL-4, the wild-type virus or controls. The data are consistent with previous findings that TK/GCV-mediated destruction of tumor cells can facilitate antigen uptake by professional antigen presenting cells. Possibly, the process of cellular destruction involves apoptosis through accumulation of p53 and the upregulation of Fas (CD-95), which then through aggregation stimulates FADD-dependent cell death in a Fas ligand independent manner (Beltinger et al. 1999, P.N.A.S. USA 96:8699-704). It is therefore plausible that VSV expressing TK exerts a greater oncolytic effect through TK/GCV mediated apoptosis and enhanced bystander effect, as well as through the generation of specific antitumor CTL responses. While IL-4 can influence the development of Th cells, IL-4 in this tumor model did not strongly influence the development of tumor specific cytotoxic T cells, as judged by CTL assays. Nevertheless, VSV-expressing IL-4 did exert a greater oncolytic effect that was statistically significant compared to wild-type VSV.

Although IL-4 has been incorporated into a number of live virus vectors for either gene therapy, anticancer strategies or to increase an immune response to candidate viral-vaccines, the overall positive effects of the cytokines contribution vary (Bebedetti et al. 2000, Nat. Med. 6:447-50). The data shown herein indicate that tumors treated with VSV expressing IL-4 may exert a more potent oncolytic effect than VSV alone possibly due to the increased presence of infiltrating eosinophils and neutrophils, which have been reported to directly have antitumor activity (Tepper et al., 1992, Science 257:548-51). However, the full mechanisms of IL-4 mediated antitumor activity undoubtedly remain to be clarified.

A recent report indicated that expression of IL-4 by ectromelia virus suppressed antiviral cell-mediated immune responses and was associated with high mortality in mice usually resistant to the wild-type virus (Jackson et al., 2001, J. Virol. 75:1205). While these data raise concerns about developing novel viruses that contain immunomodulatory genes, the data would be consistent with studies where retroviruses or adenovirus expressing IL-4 produced no adverse effects *in vivo* (Steele et al. 2000, Proc. Soc. Exp. Biol. Med. 223:118-27). In safety trials we did not note any increase in toxicity in animals inoculated, by various routes, with VSV-IL-4 compared to the wild-type VSV. Aside from not being able to detect infectious VSV in organs or tumors from mice receiving VSV treatment, 7 days after the last tumor inoculation, animals receiving VSV-IL-4 or VSV-TK at 2 x 10⁷ (i.p.) or 2 x 10⁶ (i.v.), presently remain healthy 8 weeks after infection.

### Example 5

### Materials and Methods

### Cells

BHK-21 cells, primary and transformed mouse embryonic fibroblasts derived from C57BL/6 mice were maintained in Dulbecco's modified essential medium (DMEM) supplemented with 10% fetal bovine serum (HyClone Laboratories Inc., Logan, UT), 100 units of penicillin G/ml, 100 units of streptomycin/ml and 0.25µg of amphotericin B. B16(F10) melanoma cells and DA-3 cells derived from D1-DMBA3 breast tumor were maintained in same medium except that it contained 1.5 g of sodium bicarbonate per liter and OPI media supplement (Sigma, St. Louis, MO), respectively. TS/A mammary adenocarcinoma cells were maintained RPMI 1640 medium supplemented with 10% fetal bovine serum.

### Construction of recombinant virus

Mouse IFN-β cDNA was amplified by polymerase chain reaction (PCR) from plasmid pMK-Mβ, a gift from Dr. Yoichiro Iwakura, Institute of Medical Science, University of Tokyo, using oligonucleotides 5'-TCCATCCTCGAGCACTATGAACAACAGGTGGATCCTC-3' (sense) and 5'-AGGTCTGCTAGCCTAGTTTTGGAAGTTTCTGGT-3' (anti-sense). The amplified fragment was then inserted into the Xho I-Nhe I site of pVSV-XN2 (Fernandez et al. 2002, J. Virol.; 76(2): 895-904). The procedure for recovering recombinant VSV was similar to that described previously (Lawson et al., 1995, P.N.A.S. USA, 92:4477-81; Whelan et al., 1995, P.N.A.S. USA, 92:8388-92). The seed virus was propagated in BHK-21 cells and stored at -80C until use. VSV-GFP and rVSV were prepared as previously described (Fernandez et al., 2002).

### Virus growth in vitro

The growth of the recombinant viruses in BHK-21 cells were examined as previously described (Fernandez et al., 2002). Cells were seeded in 6-well culture plate at 1x10⁶ cells per well and infected with each virus at a multiplicity of infection (m.o.i.) of 10 PFU per cell. The culture supernatants were harvested at the indicated times and subjected to titer determination by a standard plaque assay on BHK-21 cells.

For in vitro cell-killing assay, murine embryonic fibroblasts, B16(F10), DA-3, and TS/A cells were seeded in 12- or 24-well culture plate at approximately 2x10⁵ cells per well and infected with viruses at the indicated m.o.i. for 24 h, and then trypsinized and counted by trypanblue exclusion analysis.

### ELISA for IFN-β production

Expression levels of IFN-β on VSV-IFNβ-infected cells was examined by an enzyme-linked immunosorbent assay (ELISA). BHK-21 cells were seeded in a 35-mm-diameter culture dish at 1x10⁶ cells and infected with viruses at an m.o.i of 10 p.f.u. per cell for 24 h. The supernatant was then subjected to ELISA. ELISA was performed as previously published procedure (Coligan et al., 1991). Briefly, 96-well microcroplate (Nalge Nunc International, Rochester, NY) was coated with 5 µg/ml of monoclonal rat antibody to mouse IFN-β (Seikagaku America, Falmouth, MA) and incubated overnight at 4C. Serial dilutions of the supernatants were then added and incubated overnight at 4C. Polyclonal sheep antibody to mouse IFN-α/β (United States Biological, Swampscott, MA) diluted to 1: 2000 was used as secondary antibody, and bound antibodies were detected with peroxidase-labeled polyclonal rabbit antibody to sheep IgG (H+L) (KPL, Gaithersburs, MD) diluted to 1: 2500. The peroxidase was revealed by incubation with the substrate 2, 2'-azino-bis (3-ethylbenzothiazoline-6-sulfonate) for 30 min, and a spectrophotometric reading was obtained at 414 nm.

### Biological activity assay for IFN-β

BHK-21 cells were seeded in a 35-mm-diameter culture dish at 1x10⁶ cells and infected with viruses at an m.o.i of 10 p.f.u. per cell for 24 h. The supernatants were harvested and treated at 56C for 30 min to inactivate the viral infectivity. B16(F10) cells were seeded in a 24-well culture plate at 2x10⁶ cells per well and incubated with the supernatant diluted to 1: 50 or 500 units/ml of mouse IFN-α/β (Sigma) for 24 h. Cells were then infected with VSV at an m.o.i. of 0.1 for 24 h, and CPE was assessed under microscopy.

### Animal studies

Six to 8-week-old female BALB/c mice were obtained from Jackson Laboratories and maintained under specific pathogen-free conditions. Mice were injected intravenously (i.v.) with 5x10⁴ TS/A cells and then infected i.v. with 5x10⁷ p.f.u. of recombinant VSV 2 days later. The survival of mice was monitored daily after virus infection. For vaccination of VSV-IFNβ-infected TS/A cells, the cells were infected with the virus at an m.o.i. of 10 per cell for 2h. Mice were injected subcutaneously (s.c.) with 1x10⁶ infected TS/A cells and challenged sub-cutaneously with 1x10⁵ TS/A cells 10 days later. The tumor sizes were measured at 2 days intervals, and the volume was calculated according to the formula 0.5 x length x (width²).

Statistical significance of inter-group differences was evaluated using the Mann-Whitney test. For histopathological analysis, mice were killed at indicated time point, and tumors were excised, fixed with 10% neutralized buffered formalin and stained with hematoxylin and eosin.

### Cytotoxic assay

Cytotoxity of T-lymphocytes was performed as described as previously (Fernandez et al., 2002). Briefly, spleen cells prepared from VSV-infected tumor-bearing mice were incubated with ⁵¹Cr-labeled TS/A cells at indicated effecter cells to target cells ratios, and release of ⁵¹Cr was quantified on a gamma counter. The percentage of lysis was calculated according to the formula [(experimental release cpm - spontaneous release cpm)/(maximum release - spontaneous release)] x 100.

Table 4 shows the virus titers in primary and transformed C57BL/6 mouse fibroblast. Table 5 shows the virus titers in TS/A cells.

**Table 4 Virus titers in primary and transformed C57BL/6 mouse fibroblasts**

| Virus^{a)} | Virus titers (PFU/1x10^5 cells)^{b)} | |
|---|---|---|
| | Primary Cell | Transformed Cell |
| VSV-GFP | 1.3x10^7 | 3.6x10^6 |
| VSV-IFNβ | 6.2x10^4 | 2.9x10^5 |
| VSV-IFNγ | 3.5x10^6 | 1.5x10^6 |

| | | |
|---|---|---|
| ^{a)} MOI 0.01 ^{b)} Data shows a mean of twice of independent experiment | | |

**Table 5 Virus titers in TS/A cells**

| Virus | Virus titers (PFU/1x10^6 cells)^{a)} | |
|---|---|---|
| | M.O.I. 0.1 | M.O.I. 0.01 |
| VSV-GFP | 4.3x10^8 | 1.4x10^8 |
| VSV-IFNβ | 3.5x10^8 | 1.4x10^7 |
| VSV-IFNγ | 3.4x10^8 | 1.3x10^8 |

| | | |
|---|---|---|
| ^{a)} Data shows a mean of twice of independent experiment | | |

The data show that VSV-IFN-beta or IFN-gamma retain the ability to kill tumor cells. The inclusion of the IFN-beta or IFN-gamma in the VSV vector construct does not impede the VSV oncolytic activity or replicative abilities.

### Example 6

VSV Inhibits Growth of p53-Deficient, Myc-Transformed, or Ras-Transformed In Vivo.

To start to evaluate the use of VSV in antitumor therapy, athymic nude mice were subcutaneously implanted with 2 x 10⁶ C6 glioma cells, or with Balb-3T3 cells transformed with the Myc or the activated K-Ras gene. When palpable tumors had formed (approximately 7-14 days when the tumors had reached an approximate size of 0.25 cm²) mice were infected intratumorally with VSV (2.5 x 10⁷ pfu/ml) and monitored daily. The injection, with same amount of virus was repeated after four days. All mice that received VSV showed markedly-inhibited tumor growth, irrespective of the genetic backgrounds of the tumors, or the oncogenic events contributing to their transformation. In fact, the administration of VSV resulted in marked repression of tumor growth in all animals tested within 17 days, when tumors in the control animals exceeded that acceptable tumor burden. These data highlight the potent efficacy of VSV against tumors both *in vitro* and *in vivo.*

To examine whether VSV spread beyond the implanted, virus-inoculated tumor, a variety of tissue from the VSV treated animals, as well as the tumors themselves were analyzed for the presence of residual, replicating VSV. Examination of VSV infected mice for the presence of VSV 21 days after infection revealed the existence of residual virus (2 x 10⁴ -3.5 x 10⁵ pfu/g) in tumor tissue derived from C6 cells. However, no virus was detectable in the lung, brain, kidney, spleen, or liver of mice receiving VSV therapy after this period of time. These data show that VSV replication is restricted to tumor-lineage.

### Example 7

VSV Exerts Anti-Tumor Activity Intravenously and on Distal Tumors.

To examine whether VSV was capable of exerting its antitumor effects following inoculation at sites distant from the tumor, VSV was introduced intravenously (i.v; three injections of 1 x 10⁷ pfu each/mouse two days apart) and monitored growth of implanted C6 glioblastoma tumors every day for up to 8 days.

Nude mice were implanted with 1 x 10⁶ C6 cells bilaterally into both rear flanks of the mouse and the right tumor was inoculated with 1 x 10⁷ pfu VSV, or with heat-inactivated control virus. Nude mice bearing single C6 tumors were injected intravenously with 1 x 10⁸ pfu VSV at days 1, 3, 5, 7, 9, 11 and 13. i.v.-inoculated VSV was able to cause the regression of C6 tumors *in vivo.*

Next, experiments were designed to determine whether intratumoral inoculation of VSV can cause the regression of distal tumors at other sites on the mouse. For these experiments, nude mice were implanted with C6 glioblastoma cells bilaterally on both the left and right flanks of the mouse, and inoculated only one of the implanted tumors with VSV after both had reaches a size of approximately 0.25 cm². VSV, but not heat-inactivated virus control, was able to cause the partial regression of distal tumors when introduced into one tumor. These studies demonstrate the potential of VSV to eradicate tumors and metastases at sites distal from the site of inoculation.

### Example 8

### VSV from cDNA

A cDNA clone representing the entire 11,161 nucleotides of VSV was generated and unique Xho I/Nhe I sites were added to facilitate entry of a heterologous gene, in our case, HSV-TK. Transcription of the cDNA is dependent on T7 RNA polymerase. Vaccinia vTF7-3 is used to infect baby hamster kidney cells (BHK-21), to provide a source of polymerase. Subsequently, VSV cDNA is transfected into the same cells together with three other plasmids that express the VSV N, P and L proteins. These latter three proteins facilitate the assembly of nascent VSV antigenomic RNA into nucleocapsids and initiate the VSV infectious cycle. After 24 hours, host cells are lysed, clarified and residual vaccinia removed by filtration through a 0.2 um filter onto fresh BHK cells. Only recombinant VSVs are produced by this method since no wild-type VSV can be generated.

### Example 9

### Characterization of recombinant VSVs (rVSVs).

Cells infected with rVSV or wild-type VSV are metabolically labeled with [³⁵S]methionine. Cells are lysed and aliquots analyzed by SDS-PAGE. Since VSV inhibits host proteins synthesis, only viral proteins are made, including heterologous genes inserted into its genome. Cells infected with rVSVs will have an extra protein (i.e. HSV-TK, ∼26 kDa) being synthesized compared to control cells infected with VSV alone. VSV mRNAs are detected by a similar manner using radiolabeled dUTP. In many cases, antibody to the heterologous protein exists. Therefore, ELISAs are used to detect the expression of heterologous proteins, such as, IL-4, IL-12 and IFNs. High levels of heterologous protein expression have been obtained in all recombinant systems examined.

### Example 10

### Growth of VSV

Large amounts of VSV (Indiana strain) and recombinant VSV are purified by sucrose gradients. Essentially, BHK cells are infected at 0.01 m.o.i and after 24 hours, where > 80% of cells usually exhibit CPE/apoptosis, supernatants are collected and clarified by centrifugation. Clarified supernatants are purified by centrifugation through **10%** sucrose and the viral pellets resuspended and layered onto continuous 35-55% sucrose gradients. The gradients are centrifuged at 110,000g for 18 hours at 4°C and virus retrieved and pelleted by further centrifugation and 15,000 rpm at 4°C for 1 hour. Viruses are resuspended in PBS, concentrations determined by standard plaque assays and stored in aliquots at - 80°C (30).

### Example 11

### Generation of replication-defective recombinant VSV.

VSV that lacks the G protein function and which express IL-12 or IFN-β and γ are constructed. Such viruses are generated in helper cells (CHO) that have been constructed to inducibly express the VSV G protein. Following infection of target cells, resultant viruses infect cells because they contain the VSV G from the helper cell. However, following infection and replication, progeny viruses will lack the receptor G and cannot disseminate to infect surrounding tissue. It is likely that these viruses are able to infect tumor cells and preferentially replicate to express immunomodulatory or suicide protein. Replication-defective VSV viruses expressing selected heterologous genes are produced and compared regarding their anti-tumor efficacy *in vitro* and *in vivo* against wt VSV counterparts. rVSV lacking M and N protein function are produced. Additionally, VSV having a replacement of the G protein with other receptors, such as tumor specific receptors are produced and analyzed to determine if the presence of the tumor specific receptor in the rVSV is more tumor cell specific.

## Claims

1. A replication-defective recombinant vesicular stomatitis virus (VSV) vector comprising nucleic acid encoding a cytokine, wherein said recombinant VSV vector exhibits greater oncolytic activity against a tumor cell than a wild-type VSV vector when contacted with the tumor cell, wherein the cytokine is selected from the group consisting of interleukin-2, interleukin-4, interleukin-6, interleukin-12, interferon-alpha, interferon-beta, interferon-gamma, interferon-omega, interferon-epsilon, granulocyte-macrophage colony stimulating factor, and tumor necrosis factor.

2. The VSV vector of claim 1 wherein said cytokine is an interferon.

3. The VSV vector of claim 2 wherein said interferon is interferon-beta or interferon-gamma.

4. The VSV vector of claim 1 wherein said cytokine is an interleukin.

5. The VSV vector of claim 4 wherein said cytokine is IL-4 or IL-12.

6. The VSV vector of any one of the preceding claims wherein said VSV vector lacks G-protein function.

7. The VSV vector of any one of the preceding claims wherein the tumor cell is a melanoma tumor cell, mammary tumor cell, prostate tumor cell, cervical tumor cell, hematological-associated tumor cell or a cell harboring a defect in a tumor suppressor pathway.

8. The VSV vector of any one of the preceding claims wherein the tumor cell is a mammalian tumor cell.

9. The VSV vector of any one of the preceding claims further comprising nucleic acid encoding a second cytokine.

10. The VSV vector of claim 3 further comprising nucleic acid encoding an interleukin.

11. An isolated nucleic acid encoding the VSV vector of any one of the preceding claims.

12. A cell comprising the VSV vector of any of claims 1 to 10, and progeny thereof.

13. A method of producing a VSV comprising nucleic acid encoding a cytokine comprising, growing a cell according to claim 12 under conditions whereby VSV is produced; and optionally isolating said VSV.

14. A recombinant vesicular stomatitis viral particle comprising the VSV vector of any one of claims 1 to 10.

15. A composition comprising the VSV vector of any one of claims 1 to 10 and a pharmaceutically acceptable excipient.

16. The composition of claim 15 wherein said VSV vector is present in the composition in an amount effective to produce oncolytic activity of a tumor cell when said composition is contacted with the tumor cell.

17. Use of the VSV vector of any one of claims 1 to 10 or the composition of claim 15 or 16 in the preparation of a medicament for treating a tumor.

18. Use of a replication-defective recombinant VSV vector comprising nucleic acid encoding a suicide gene wherein said VSV vector exhibits greater tumor suppression or greater oncolytic activity when administered along with a prodrug than a wild-type VSV vector in the preparation of a medicament for treating a tumor, wherein the prodrug is selected from the group consisting of 5-fluorocytosine, ganciclovir and acyclovir.

19. The use of claim 18 wherein said suicide gene encodes thymidine kinase (TK).

20. The use of claim 19 wherein said prodrug is ganciclovir or acyclovir.

21. The use of any one of claims 18 to 20 wherein said VSV vector lacks G-protein.

22. The use of any one of claims 17 to 21 wherein the tumor cell is a melanoma tumor, mammary tumor, prostate tumor, cervical tumor, hematological-associated tumor or tumor comprising cells harboring a defect in a tumor suppressor pathway.

23. The use of any one of claims 17 to 22 wherein said medicament is for administration by intravenous or intratumoral injection.

24. Use of tumor cells infected with or lysed by a VSV vector comprising nucleic acid encoding a cytokine, chemokine or heat shock protein in the manufacture of a medicament for treating a tumor, wherein the cytokine is selected from the group consisting of interleukin-2, interleukin-4, interleukin-6, interleukin-12, interferon-alpha, interferon-beta, interferon-gamma, interferon-omega, interferon-epsilon, granulocyte-macrophage colony stimulating factor, and tumor necrosis factor.

25. The use of claim 24 wherein the cytokine is an interferon or interleukin.

26. A composition for use in inducing an immune response in an individual comprising a pharmaceutically acceptable excipient and tumor cells infected with or lysed by a VSV vector comprising nucleic acid encoding a cytokine, chemokine or heat shock protein, wherein the cytokine is selected from the group consisting of interleukin-2, interleukin-4, interleukin-6, interleukin-12, interferon-alpha, interferon-beta, interferon-gamma, interferon-omega, interferon-epsilon, granulocyte-macrophage colony stimulating factor, and tumor necrosis factor.

27. A method for preparing the composition of claim 26 comprising, contacting a tumor cell obtained from an individual with a VSV vector comprising nucleic acid encoding a cytokine, chemokine or heat shock protein under conditions suitable for lysing said tumor cells, wherein the cytokine is selected from the group consisting of interleukin-2, interleukin-4, interleukin-6, interleukin-12, interferon-alpha, interferon-beta, interferon-gamma, interferon-omega, interferon-epsilon, granulocyte-macrophage colony stimulating factor, and tumor necrosis factor.

## Patentansprüche

1. Replikationsdefekter rekombinanter vesikulärer Stomatitis-Virus-Vektor (vesicular stomatitis virus, VSV), umfassend Nukleinsäure, die für ein Zytokin kodiert, wobei der rekombinante VSV-Vektor eine größere onkolytische Aktivität gegen eine Tumorzelle aufweist als ein Wildtyp-VSV-Vektor, wenn er mit der Tumorzelle in Kontakt kommt, wobei das Zytokin aus der Gruppe bestehend aus Interleukin-2, Interleukin-4, Interleukin-6, Interleukin-12, Interferon-alpha, Interferon-beta, Interferon-gamma, Interferon-omega, Interferon-epsilon, Granulozyten-Makrophagen-Kolonien stimulierendem Faktor und Tumornekrosefaktor ausgewählt ist.

2. VSV-Vektor nach Anspruch 1, wobei das Zytokin ein Interferon ist.

3. VSV-Vektor nach Anspruch 2, wobei das Interferon Interferon-beta oder Interferon-gamma ist.

4. VSV-Vektor nach Anspruch 1, wobei das Zytokin ein Interleukin ist.

5. VSV-Vektor nach Anspruch 4, wobei das Zytokin IL-4 oder IL-12 ist.

6. VSV-Vektor nach einem der vorstehenden Ansprüche, wobei dem VSV-Vektor die G-Protein-Funktion fehlt.

7. VSV-Vektor nach einem der vorstehenden Ansprüche, wobei die Tumorzelle eine Melanomtumorzelle, Mammatumorzelle, Prostatatumorzelle, Gebärmutterhalstumorzelle, hämatologisch verbundene Tumorzelle oder eine Zelle ist, die einen Defekt in einem Tumorsuppressorpfad beherbergt.

8. VSV-Vektor nach einem der vorstehenden Ansprüche, wobei die Tumorzelle eine Säugetier-Tumorzelle ist.

9. VSV-Vektor nach einem der vorstehenden Ansprüche, ferner umfassend Nukleinsäure, die für ein zweites Zytokin kodiert.

10. VSV-Vektor nach Anspruch 3, ferner umfassend Nukleinsäure, die für ein Interleukin kodiert.

11. Isolierte Nukleinsäure, die für den VSV-Vektor nach einem der vorstehenden Ansprüche kodiert.

12. Zelle, umfassend den VSV-Vektor nach einem der Ansprüche 1 bis 10 und Nachkommen davon.

13. Verfahren zum Produzieren eines VSV, umfassend Nukleinsäure, die für ein Zytokin kodiert, umfassend ein Züchten einer Zelle gemäß Anspruch 12 unter Bedingungen, mittels derer VSV produziert wird; und optional das Isolieren des VSV.

14. Rekombinantes vesikuläres Stomatitis-Viruspartikel, umfassend den VSV-Vektor nach einem der Ansprüche 1 bis 10.

15. Zusammensetzung, umfassend den VSV-Vektor nach einem der Ansprüche 1 bis 10 und einen pharmazeutisch annehmbaren Trägerstoff.

16. Zusammensetzung nach Anspruch 15, wobei der VSV-Vektor in der Zusammensetzung in einer Menge vorhanden ist, die zum Produzieren onkolytischer Aktivität einer Tumorzelle wirksam ist, wenn die Zusammensetzung mit der Tumorzelle in Kontakt kommt.

17. Verwendung des VSV-Vektors nach einem der Ansprüche 1 bis 10 oder der Zusammensetzung nach Anspruch 15 oder 16 bei der Produktion eines Medikaments zum Behandeln eines Tumors.

18. Verwendung eines replikationsdefekten rekombinanten VSV-Vektors, umfassend Nukleinsäure, der für ein Suizidgen kodiert, wobei der VSV-Vektor bei der Produktion eines Medikaments zum Behandeln eines Tumors eine größere Tumorsuppression oder einer größere onkolytische Aktivität aufweist als ein Wildtyp-VSV-Vektor, wenn er zusammen mit einem Prodrug verabreicht wird, wobei das Prodrug aus der Gruppe bestehend aus 5-Fluorcytosin, Ganciclovir und Acyclovir ausgewählt ist.

19. Verwendung nach Anspruch 18, wobei das Suizidgen für Thymidinkinase (TK) kodiert.

20. Verwendung nach Anspruch 19, wobei das Prodrug Ganciclovir oder Acyclovir ist.

21. Verwendung nach einem der Ansprüche 18 bis 20, wobei dem VSV-Vektor G-Protein fehlt.

22. Verwendung nach einem der Ansprüche 17 bis 21, wobei die Tumorzelle ein Melanomtumor, Mammatumor, Prostatatumor, Gebärmutterhalstumor, hämatologisch verbundener Tumor oder ein Tumor, der Zellen, die einen Defekt in einem Tumorsuppressorpfad beherbergen, umfasst, ist.

23. Verwendung nach einem der Ansprüche 17 bis 22, wobei das Medikament zur Verabreichung durch intravenöse oder intratumorale Injektion ist.

24. Verwendung von Tumorzellen, die durch einen VSV-Vektor infiziert oder lysiert sind, der Nukleinsäure umfasst, die für ein Zytokin, Chemokin oder ein Hitzeschockprotein kodiert, bei der Herstellung eines Medikaments zum Behandeln eines Tumors, wobei das Zytokin aus der Gruppe bestehend aus Interleukin-2, Interleukin-4, Interleukin-6, Interleukin-12, Interferon-alpha, Interferon-beta, Interferon-gamma, Interferon-omega, Interferon-epsilon, Granulozyten-Makrophagen-Kolonien stimulierendem Faktor und Tumornekrosefaktor ausgewählt ist.

25. Verwendung nach Anspruch 24, wobei das Zytokin ein Interferon oder Interleukin ist.

26. Zusammensetzung zur Verwendung beim Induzieren einer Immunreaktion in einem Individuum, umfassend einen pharmazeutisch annehmbaren Trägerstoff und Tumorzellen, die durch einen VSV-Vektor infiziert oder lysiert sind, der Nukleinsäure umfasst, die für ein Zytokin, Chemokin oder ein Hitzeschockprotein kodiert, wobei das Zytokin aus der Gruppe bestehend aus Interleukin-2, Interleukin-4, Interleukin-6, Interleukin-12, Interferon-alpha, Interferon-beta, Interferon-gamma, Interferon-omega, Interferon-epsilon, Granulozyten-Makrophagen-Kolonien stimulierendem Faktor und Tumornekrosefaktor ausgewählt ist.

27. Verfahren zum Herstellen der Zusammensetzung nach Anspruch 26, umfassend das Kontaktieren einer Tumorzelle, die von einem Individuum erhalten wurde, mit einem VSV-Vektor, umfassend Nukleinsäure, die für ein Zytokin, Chemokin oder Hitzeschockprotein kodiert, unter Bedingungen, die zum Lysieren der Tumorzellen geeignet sind, wobei das Zytokin aus der Gruppe bestehend aus Interleukin-2, Interleukin-4, Interleukin-6, Interleukin-12, Interferon-alpha, Interferon-beta, Interferon-gamma, Interferon-omega, Interferon-epsilon, Granulozyten-Makrophagen-Kolonien stimulierendem Faktor und Tumornekrosefaktor ausgewählt ist.

## Revendications

1. Vecteur du virus de stomatite vésiculaire (VSV) recombinant non réplicatif comprenant un acide nucléique codant pour une cytokine, ledit vecteur du VSV recombinant présentant une activité oncolytique contre une cellule tumorale supérieure à un vecteur du VSV de type sauvage lorsqu'il est mis en contact avec la cellule tumorale, la cytokine étant choisie dans le groupe constitué par l'interleukine 2, l'interleukine 4, l'interleukine 6, l'interleukine 12, l'interféron alpha, l'interféron bêta, l'interféron gamma, l'interféron oméga, l'interféron epsilon, un facteur de stimulation des colonies de granulocytes-macrophages et un facteur de nécrose tumorale.

2. Vecteur du VSV de la revendication 1, dans lequel ladite cytokine est un interféron.

3. Vecteur du VSV de la revendication 2, dans lequel ledit interféron est l'interféron bêta ou l'interféron gamma.

4. Vecteur du VSV de la revendication 1, dans lequel ladite cytokine est une interleukine.

5. Vecteur du VSV de la revendication 4, dans lequel ladite cytokine est IL-4 ou IL-2.

6. Vecteur du VSV de l'une quelconque des revendications précédentes, ledit vecteur du VSV étant dépourvu de fonction de protéine G.

7. Vecteur du VSV de l'une quelconque des revendications précédentes, dans lequel la cellule tumorale est une cellule tumorale de mélanome, une cellule tumorale mammaire, une cellule tumorale prostatique, une cellule tumorale du col de l'utérus, une cellule tumorale hématologique ou une cellule porteuse d'un défaut dans une voie de suppresseur tumoral.

8. Vecteur du VSV de l'une quelconque des revendications précédentes, dans lequel la cellule tumorale est une cellule tumorale de mammifère.

9. Vecteur du VSV de l'une quelconque des revendications précédentes, comprenant en outre un acide nucléique codant pour une deuxième cytokine.

10. Vecteur du VSV de la revendication 3, comprenant en outre un acide nucléique codant pour une interleukine.

11. Acide nucléique isolé codant pour le vecteur du VSV de l'une quelconque des revendications précédentes.

12. Cellule comprenant le vecteur du VSV de l'une quelconque des revendications 1 à 10 et sa progéniture.

13. Procédé de production d'un VSV comprenant un acide nucléique codant pour une cytokine, comprenant la croissance d'une cellule selon la revendication 12 dans des conditions grâce auxquelles le VSV est produit ; et éventuellement l'isolement dudit VSV.

14. Particule virale de la stomatite vésiculaire recombinante comprenant le vecteur du VSV de l'une quelconque des revendications 1 à 10.

15. Composition comprenant le vecteur du VSV de l'une quelconque des revendications 1 à 10 et un excipient pharmaceutiquement acceptable.

16. Composition de la revendication 15, ledit vecteur du VSV étant présent dans la composition dans une quantité efficace pour produire une activité oncolytique d'une cellule tumorale lorsque ladite composition est mise en contact avec la cellule tumorale.

17. Utilisation du vecteur du VSV de l'une quelconque des revendications 1 à 10 ou de la composition de la revendication 15 ou 16 dans la préparation d'un médicament destiné à traiter une tumeur.

18. Utilisation d'un vecteur du VSV recombinant non réplicatif comprenant un acide nucléique codant pour un gène suicide, ledit vecteur du VSV présentant une activité de suppression tumorale ou une activité oncolytique, lorsqu'il est administré avec un promédicament, supérieure à un vecteur du VSV de type sauvage dans la préparation d'un médicament destiné à traiter une tumeur, le promédicament étant choisi dans le groupe constitué par la 5-fluorocytosine, le ganciclovir et l'acyclovir.

19. Utilisation de la revendication 18, dans laquelle ledit gène suicide code pour une thymidine kinase (TK).

20. Utilisation de la revendication 19, dans laquelle ledit promédicament est le ganciclovir ou l'acyclovir.

21. Utilisation de l'une quelconque des revendications 18 à 20, dans laquelle ledit vecteur du VSV est dépourvu de protéine G.

22. Utilisation de l'une quelconque des revendications 17 à 21, dans laquelle la cellule tumorale est une tumeur de mélanome, une tumeur mammaire, une tumeur prostatique, une tumeur du col de l'utérus, une tumeur hématologique ou une tumeur comprenant des cellules porteuses d'un défaut dans une voie de suppresseur tumoral.

23. Utilisation de l'une quelconque des revendications 17 à 22, dans laquelle ledit médicament est destiné à une administration par voie intraveineuse ou par injection intratumorale.

24. Utilisation de cellules tumorales infectées ou lysées par un vecteur du VSV comprenant un acide nucléique codant pour une cytokine, une chimiokine ou une protéine de choc thermique dans la fabrication d'un médicament destiné à traiter une tumeur, la cytokine étant choisie dans le groupe constitué par l'interleukine 2, l'interleukine 4, l'interleukine 6, l'interleukine 12, l'interféron alpha, l'interféron bêta, l'interféron gamma, l'interféron oméga, l'interféron epsilon, un facteur de stimulation des colonies de granulocytes-macrophages et un facteur de nécrose tumorale.

25. Utilisation de la revendication 24, dans laquelle la cytokine est un interféron ou une interleukine.

26. Composition destinée à une utilisation visant à induire une réponse immunitaire chez un individu, comprenant un excipient pharmaceutiquement acceptable et des cellules tumorales infectées ou lysées par un vecteur du VSV comprenant un acide nucléique codant pour une cytokine, une chimiokine ou une protéine de choc thermique, la cytokine étant choisie dans le groupe constitué par l'interleukine 2, l'interleukine 4, l'interleukine 6, l'interleukine 12, l'interféron alpha, l'interféron bêta, l'interféron gamma, l'interféron oméga, l'interféron epsilon, un facteur de stimulation des colonies de granulocytes-macrophages et un facteur de nécrose tumorale.

27. Procédé de préparation de la composition de la revendication 26, comprenant la mise en contact d'une cellule tumorale prélevée sur un individu avec un vecteur du VSV comprenant un acide nucléique codant pour une cytokine, une chimiokine ou une protéine de choc thermique dans des conditions appropriées pour lyser lesdites cellules tumorales, la cytokine étant choisie dans le groupe constitué par l'interleukine 2, l'interleukine 4, l'interleukine 6, l'interleukine 12, l'interféron alpha, l'interféron bêta, l'interféron gamma, l'interféron oméga, l'interféron epsilon, un facteur de stimulation des colonies de granulocytes-macrophages et un facteur de nécrose tumorale.
